# EUROPEAN PATENT APPLICATION

(11) **EP 2 735 569 A1**
(43) Date of publication of application: **28.05.2014**
(21) Application number: 12805327.9
(22) Date of filing: 28.06.2012
(51) Int. Cl.: C07K 5/06, A23L 1/226, C07D 281/06

(54) **METHOD FOR PRODUCING LANTHIONINE DERIVATIVE**

(30) Priority: 28.06.2011 JP 2011142890
(71) Applicant: Ajinomoto Co., Inc., Kanagawa 210-8681 (JP)
(72) Inventor: KATO Yumiko, Kawasaki-shi Kanagawa 210-8681 (JP); MIZUKOSHI Toshimi, Kawasaki-shi Kanagawa 210-8681 (JP); SUZUKI Yumiko, Kawasaki-shi Kanagawa 210-8681 (JP); SATO Seiichi, Kawasaki-shi Kanagawa 210-8681 (JP)
(74) Representative: Strehl Schübel-Hopf & Partner
(86) International application number: PCT/JP2012/066534
(87) International publication number: WO 2013/002329

(57) **Abstract**

The present invention relates to a method for producing a novel lanthionine derivative having a CaSR agonist activity, an intermediate compound of the lanthionine derivative, and use of the intermediate compound for production of a CaSR agonist, a kokumi-imparting agent, and a food and/or beverage ingredient.

## Description

### Technical Field

The present invention relates to a method for producing a novel lanthionine derivative having a CaSR agonist activity, an intermediate compound of the lanthionine derivative, and use of the intermediate compound for production of a CaSR agonist, a kokumi-imparting agent, or a food and/or beverage ingredient.

### Background Art

The consumers' demands for the sense of taste have recently been increased due to, for instance, the diversity of the eating habits. In this situation, there is a growing need for the excellent kokumi-imparting agent capable of imparting "kokumi". Kokumi is a sense of taste in which a marginal taste(s) of a basic taste(s) is(are) enhanced. The marginal tastes include the thickness, the growth (mouthfulness), the continuity and the harmony which cannot be expressed by the five basic tastes, namely the sweet taste, the salty taste, the sour taste, the bitter taste and the umami taste (savoriness).

Meanwhile, the calcium sensing receptor (CaSR) is also called the calcium receptor, the receptor signals of it can control a variety of biological functions within the living bodies, and the substance possessing such a CaSR agonist activity can be used as a kokumi-imparting agent (Patent Literature 1, Non Patent Literature 1).

In addition, glutathione has been known from the past as a compound having a kokumi-imparting activity. However, glutathione contains cysteine, which is a sulfur atom-containing amino acid, in its molecule, and hence has problems of the stability, odor, and the like.

Accordingly, there is a demand for provision of a method for producing a novel lanthionine derivative having a CaSR agonist activity, an intermediate compound of the lanthionine derivative, and use of the intermediate compound for production of a CaSR agonist, a kokumi-imparting agent, and a food and/or beverage ingredient.

Non Patent Literature 2 discloses a method for synthesizing a compound represented by the following formula A.

Non Patent Literature 3 discloses a reaction for converting a cystine derivative to a lanthionine derivative. Non Patent Literature 4 discloses reactions of peptide derivatives including serine sulfuric acid ester.

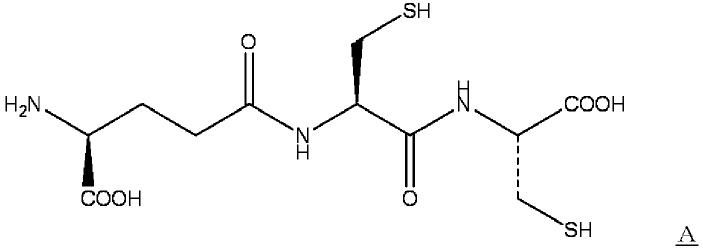

### Citation List

### Patent Literatures

Patent Literature 1: International Publication No. W02007/055393
Patent Literature 2: Japanese Patent Application Publication No. 2011-115186

### Non Patent Literatures

Non Patent Literature 1: The Journal of Biological Chemistry (2010), 285 (2), 1016-22
Non Patent Literature 2: Amino Acids (1999), 17, 257-265
Non Patent Literature 3: J. Chem. Soc., Chem. Commun., 1991 903-904
Non Patent Literature 4: J. Chem. Soc., Perkintrans 1, 2002 682-686

### Summary of Invention

### Technical Problems

An object of the present invention is to provide a method for producing a novel lanthionine derivative having a CaSR agonist activity, an intermediate compound of the lanthionine derivative, and use of the intermediate compound for production of a CaSR agonist, a kokumi-imparting agent, or a food and/or beverage ingredient.

The present inventors have diligently tested a variety of compounds so far, and have consequently found that a novel compound, lanthionine derivative, has a high CaSR agonist activity and an extremely excellent kokumi-imparting effect, and that addition of the newly found compound makes it possible to obtain a favorable food and/or beverage ingredient having an enhanced kokumi. Here, the present inventors have acquired new knowledge about a method for producing the novel lanthionine derivative, an intermediate compound of the lanthionine derivative, and use of the intermediate compound for production of a CaSR agonist, a kokumi-imparting agent, and a food and/or beverage ingredient, and completed the present invention.

### Solution to Problems

Specifically, the present invention provides a method for producing a compound represented by formula (II) (hereinafter also referred to as "compound (II)") or a chemically acceptable salt thereof, the method comprising a step of converting, by a reaction under an alkaline condition, a compound represented by the following general formula (I) or a chemically acceptable salt thereof to the compound (II) or the chemically acceptable salt thereof: 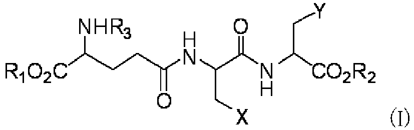 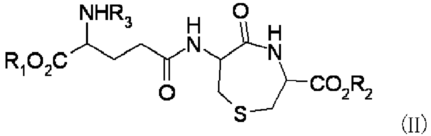
wherein R₁ and R₂, which may be the same or different, each represents a hydrogen atom or an optionally substituted lower alkyl group,
R₃ represents a hydrogen atom, an optionally substituted alkoxycarbonyl group, an optionally substituted sulfonyl group, or an acyl group,
X represents SR₄, OR₅, or a halogen atom, and Y represents SR₆, OR₇, or a halogen atom, provided that X and Y may be the same or different and at least one of X and Y is SR₄ or SR₆,
R₄ and R₆, which may be the same or different, each represents a hydrogen atom, an acyl group, an optionally substituted lower alkyl group, PO₃H₂, or SO₃H, and
R₅ and R₇, which may be the same or different, each represents an acyl group, an optionally substituted lower alkyl group, PO₃H₂, or SO₃H.

Moreover, the present invention provides a method for producing a compound represented by formula (II) or a chemically acceptable salt thereof, the method comprising a step of converting, by a cyclization reaction, a compound represented by the following general formula (I-2) or a chemically acceptable salt thereof to the compound (II) or the chemically acceptable salt thereof: 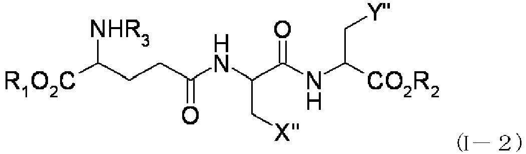 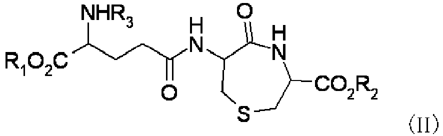
wherein R₁ and R₂, which may be the same or different, each represents a hydrogen atom or an optionally substituted lower alkyl group,
R₃ represents a hydrogen atom, an optionally substituted alkoxycarbonyl group, an optionally substituted sulfonyl group, or an acyl group,
X" and Y" each represents SH, SR₄, OR₅, or a halogen atom, provided that one of X" and Y" is SH, and the other one is not SH, and
R₄ and R₅, which may be the same or different, each represents an acyl group, an optionally substituted lower alkyl group, PO₃H₂, or SO₃H.

Further, the present invention provides a method for producing compound (III) or a chemically acceptable salt thereof, the method comprising converting, by a reaction under an alkaline condition, a compound represented by the following formula (IV) or a chemically acceptable salt thereof to the compound (III) or a compound represented by the chemically acceptable salt thereof:

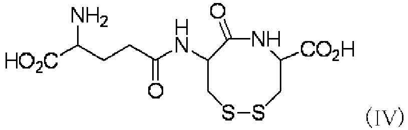

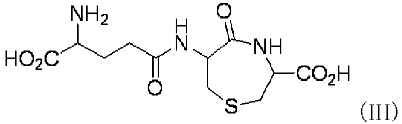

Moreover, the present invention provides a method for producing a compound represented by formula (III) or a chemically acceptable salt thereof in a case where R₁, R₂ and/or R₃ in the compound represented by formula (II) are/is a group (s) other than a hydrogen atom, the method comprising a step of removing a protective group(s), which is(are) the group(s) other than a hydrogen atom, from the compound represented by formula (II):

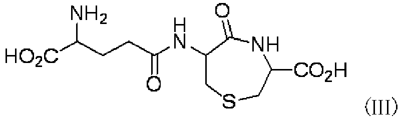

Furthermore, the present invention also provides a compound represented by the following formula (V) or a chemically acceptable salt thereof: 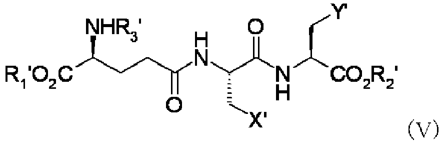
wherein R₁' and R₂', which may be the same or different, each represents a hydrogen atom or an optionally substituted lower alkyl group,
R₃' represents a hydrogen atom, an optionally substituted alkoxycarbonyl group, or an optionally substituted sulfonyl group, and
one of X' and Y' is SH, and the other one is OR₅', where R₅' represents a hydrogen atom, an acyl group, an optionally substituted lower alkyl group, PO₃H₂, or SO₃H, provided that when X' is SH, Y' is not OH.

Further, the present invention also provides use of the compound represented by formula (V) or a chemically acceptable salt thereof for production of a CaSR agonist, a kokumi-imparting agent, or a food and/or beverage ingredient.

### Description of Embodiments

Hereinafter, the present invention is described in detail.

In the present invention, "optionally substituted" means that the substituent or atom modified by this term may have one or multiple substituents. The substituents may be any ones of ordinary substituents commonly used in this field. Examples of the substituents include halogen atoms, lower alkyl, halogenated lower alkyl, lower alkenyl, and aryl groups, a hydroxyl group, a methoxy group, an ethoxy group, an acetoxy group, a carboxyl group, a nitro group, an amino group, and the like, unless otherwise noted. The substituents are each preferably a lower alkyl, lower alkenyl, or aryl group.

In the present invention, the "lower alkyl group" means a linear, branched, or cyclic alkyl group having 1 to 6 carbon atoms, and specific examples thereof include a methyl group, an ethyl group, a n-propyl group, an isopropyl group, a n-butyl group, an isobutyl group, a t-butyl group, and the like. The lower alkyl group is preferably a methyl group, an ethyl group, or the like.

The "alkoxycarbonyl group" means an alkyl-O-(CO)-group, where the alkyl is any of the above-described alkyl groups having 1 to 6 carbon atoms, such as methyl, ethyl, propyl, and t-butyl groups. The alkoxycarbonyl group is particularly preferably a Boc group. In addition, if the alkoxycarbonyl group has a substituent(s), preferred examples of the substituent(s) include a phenyl group, a 9H-fluorenyl group, and the like, in addition to the above-described substituents.

The "acyl group" means alkyl-O-, where the alkyl is the same as described above. In addition, if the acyl group has a substituent(s), preferred examples of the substituent(s) include C₁₋₃ alkyl groups and the like, in addition to the above-described substituents.

In the present invention, the following are preferable in general formula (I) or (II):
X is SR₄, and Y is OR₇;
X is OR₅, and Y is SR₆;
X is SR₄, and Y is SR₆;
X is SR₄, Y is SR₆, and R₄ and R₆ are SO₃H;
X and Y, which may be the same or different, each represents SH or OH, provided that at least one of X and Y represents SH;
X is SR₄, and Y is SR₆, where one of R₄ and R₆ is a hydrogen atom; and
X is SR₄, and Y is SR₆, where R₄ and R₆ are both hydrogen atoms.

In the present invention, the following are particularly preferable in general formula (I) or (II):
X is SR₄, and Y is SR₆, where R₄ and R₆ are SO₃H, and R₁, R₂, and R₃ are all hydrogen atoms. Note that the present invention also includes compounds (oxidative dimers) in which two molecules of a compound(s) represented by general formula (I) or (I-2) in which X' or Y' is SH are dimerized via a disulfide bond.

Regarding the three asymmetric carbons in general formula (II), the compound may have any configuration. However, the configuration shown in the following formula (II-1) is preferable:

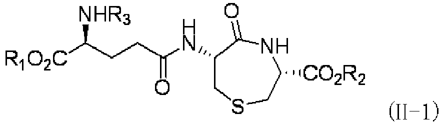

In the present invention, specific examples of the "chemically acceptable salt thereof" include ammonium salt; salts with alkali metals such as sodium and potassium; salts with alkaline earth metals such as calcium and magnesium; aluminum salt; zinc salt; organic amine salts such as triethylamine, ethanolamine, morpholine, pyrrolidine, piperidine, piperazine, dicyclohexylamine, and tetrabutylammonium salts; salts with basic amino acids such as arginine, and lysine, in the case of acidic groups such as a carboxyl group. Meanwhile, in the case of basic groups, specific examples of the "chemically acceptable salt thereof" include salts with inorganic acids such as hydrochloric acid, sulfuric acid, phosphoric acid, nitric acid, and hydrobromic acid; salts with organic carboxylic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, teoclic acid, salicylic acid, lactic acid, oxalic acid, mandelic acid, and malic acid; and salts with organic sulfonic acids such as methanesulfonic acid, benzenesulfonic acid, and p-toluenesulfonic acid.

The method for producing compound (II) or a chemically acceptable salt thereof of the present invention can be achieved by, for example, an approach in the field of organic synthetic chemistry.

A solvent used in the method for producing compound (II) or a chemically acceptable salt thereof of the present invention is not particularly limited, as long as the solvent is commonly used. It is preferable to use an aqueous solvent. In addition, in the method for producing compound (II) or a chemically acceptable salt thereof of the present invention, the "reaction under an alkaline condition" means that the reaction is carried out with the pH of the reaction liquid being adjusted to an alkaline region with an alkali such as sodium hydroxide, potassium hydroxide, or triethylamine. The pH of the alkaline reaction liquid is generally 8.0 to 14, preferably 9.0 to 13, and more preferably 9.5 to 12.5. In addition, the reaction may be conducted at room temperature, and it is also preferable to conduct the reaction under heating. Particularly when X and Y in general formula (I) are each SH or OH, it is preferable to conduct the heating. For the heating, the temperature and the heating time are not particularly limited, as long as the temperature and the heating time are commonly employed in this field. The temperature is preferably 40°C to 100°C, and more preferably in a temperature range from 60°C to 100°C, and further more preferably in a temperature range from 80°C to 100°C. Meanwhile, the heating time is preferably 1 hour to 20 hours, more preferably 2 hours to 12 hours, and further more preferably 3 hours to 8 hours.

In the step of converting, by a cyclization reaction, the compound represented by formula (I-2) of the present invention or a chemically acceptable salt thereof to compound (II) or a chemically acceptable salt thereof, the pH of the reaction liquid is not particularly limited, and is generally 8.0 to 14, preferably 9.0 to 13, and more preferably 9.5 to 12.5. In addition, the solvent used here is not particularly limited, as long as the solvent is commonly used. The solvent includes water, methanol, ethanol, isopropanol, dioxane, tetrahydrofuran, toluene, and the like. The temperature is preferably 0°C to 100°C, and more preferably in a temperature range from 40°C to 100°C, and further more preferably in a temperature range from 80°C to 100°C. In formula (I-2), groups preferably represented by R₁, R₂, and R₃ include the groups preferably represented by R₁, R₂, and R₃ in formula (I). Meanwhile, groups preferable as X" include a thiol group, a phosphonyloxy group, a sulfonyloxy group, a methanesulfonyloxy group, a chlorine atom, a bromine atom, an iodine atom, and the like. Groups preferable as Y" include a thiol group, a phosphonyloxy group, a sulfonyloxy group, a methanesulfonyloxy group, a chlorine atom, a bromine atom, an iodine atom, and the like.

In addition, in a reaction under heating of a compound in which R₁, R₂, and R₃ are hydrogen atoms, and Xw" and Y" are thiol groups, it is preferable to conduct heating under a weak acidic condition prior to the heating under the above-described alkaline condition. The temperature is not particularly limited, and is preferably 40°C to 100°C, more preferably in a temperature range from 60°C to 100°C, and further more preferably in a temperature range from 60°C to 80°C. The pH of the reaction liquid is generally 3 to 6, preferably 3.5 to 5.5, and more preferably 4.0 to 5.0. Moreover, it is preferable to add a divalent or trivalent metal salt at an initial stage of the reaction under heating. The metal salt is more preferably an iron, calcium, magnesium, or zinc salt. When a metal salt is added, it is preferable to add a compound having a chelating ability or the like during the reaction under heating, and the compound having a chelating ability includes EDTA (ethylenediaminetetraacetic acid), EGTA (glycol ether diamine tetraacetic acid), citric acid, various chelating resins, and the like. Thus, any methods having the heating step under the alkaline condition are included in the production method of the present invention, even when the methods have a heating step under a weak acidic condition as an additional step.

The solvent used in the step of converting the compound represented by formula (IV) of the present invention or a chemically acceptable salt thereof to the compound represented by compound (III) is not particularly limited, and it is preferable to use an aqueous solvent.

The step of converting the compound represented by formula (IV) of the present invention or a chemically acceptable salt thereof to the compound represented by compound (III) can be conducted according to the method described in Non Patent Literature 3. Here, the "reaction under an alkaline condition" means that the reaction is carried out with the pH of the reaction liquid being adjusted to an alkaline region with an alkali such as sodium hydroxide, potassium hydroxide, or triethylamine. The pH of the alkaline reaction liquid is generally 8.0 to 14, preferably 9.0 to 13, and more preferably 9.5 to 12. In addition, the reaction may be conducted at room temperature, and it is also preferable to conduct heating. For the heating, the temperature and the heating time are not particularly limited, as long as the temperature and the heating time are commonly employed in this field. The temperature is preferably 40°C to 100°C, more preferably in a temperature range from 60°C to 100°C, and further more preferably in a temperature range from 80°C to 100°C.

Meanwhile, the heating time is preferably 1 hour to 20 hours, more preferably 2 hours to 12 hours, and further more preferably 3 hours to 8 hours. In addition, a thiol derivative such as dithiothreitol (DTT) or a phosphine derivative such as tris(2-carboxyethyl)phosphine (TCEP) may be added during the reaction under heating.

In the method for producing a compound represented by formula (III) or a chemically acceptable salt thereof in a case where R₁, R₂ and/or R₃ in the compound represented by formula (II) are/is a group(s) other than a hydrogen atom, the method comprising a step of removing a protective group(s), which is(are) the group(s) other than a hydrogen atom, from the compound represented by formula (II), a method described in Protective Groups in Organic Synthesis, the third edition (written by T. W. Green, P. G. M. Wuts, published by JOHN WILLY & SONS, INC.) can be employed, as appropriate, for the deprotection.

In addition, the method for producing compound (II) or a chemically acceptable salt thereof of the present invention can also be achieved by treating, for example, a food and/or beverage ingredient containing a suitable starting material. For example, the lanthionine derivative can also be obtained in a reaction system by conducting a heat treatment on a microorganism culture liquid containing γ-Glu-Cys-Cys, γ-Glu-Ser-Cys, a derivative thereof, or the like. This method is also an embodiment of the present invention.

Compound (I) used in the method of the present invention (including the compounds (I-2) and (V) of the present invention, hereinafter also referred simply as a compound (I) of the present invention) or a chemically acceptable salt thereof can be produced by, for example, an approach in the field of organic synthetic chemistry.

For example, compound (I) in which X is an optionally substituted oxygen atom and Y is an optionally substituted sulfur atom can be synthesized according to the following synthetic scheme I. 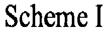 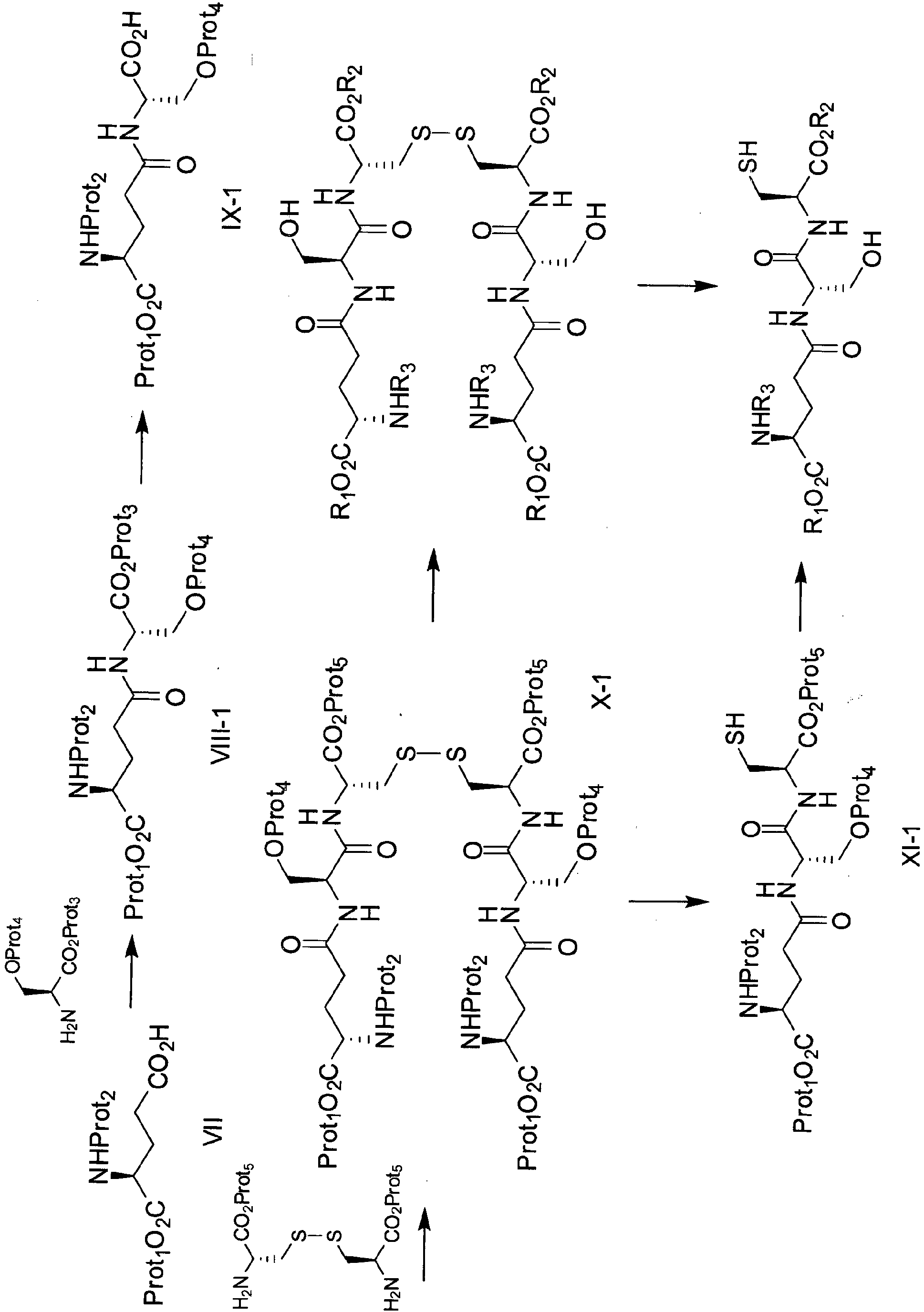 wherein Prot1 to Prot5 each independently represents a suitable protective group.

Specifically, first, dipeptide (VIII-1) is obtained from glutamic acid derivative (VII) and a serine derivative by using a commonly used condensation agent. Next, carboxylic acid (IX-1) obtained by hydrolysis of compound (VIII-1) and a cystine derivative are condensed to prepare tripeptide (X-1). Direct deprotection of tripeptide (X-1) makes it possible to obtain oxidatively dimerized compound (I). On the other hand, thiol compound (XI-1) can be obtained by reducing the disulfide bond of compound (X-1), for example, with a phosphine derivative, and reduced compound (I) is obtained by removing the protective groups of compound (XI-1). The reduced compound can also be obtained by reducing oxidized compound (I) with a phosphine derivative as in the above-described case. The thus obtained compounds represented by general formula (I) can be isolated and purified by known separation **techniques** such as **concentration under reduced pressure,** solvent extraction, crystallization, and chromatography.

In addition, as an example of compound (I) or (V), a compound (including an oxidative dimer thereof) in which R₁, R₂, and R₃ (R₁', R₂', and R₃') are hydrogen atoms, X (X') is a hydroxy group, and Y (Y') is a thiol group can be produced, for example, according to the following synthetic scheme II. 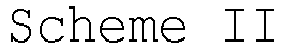 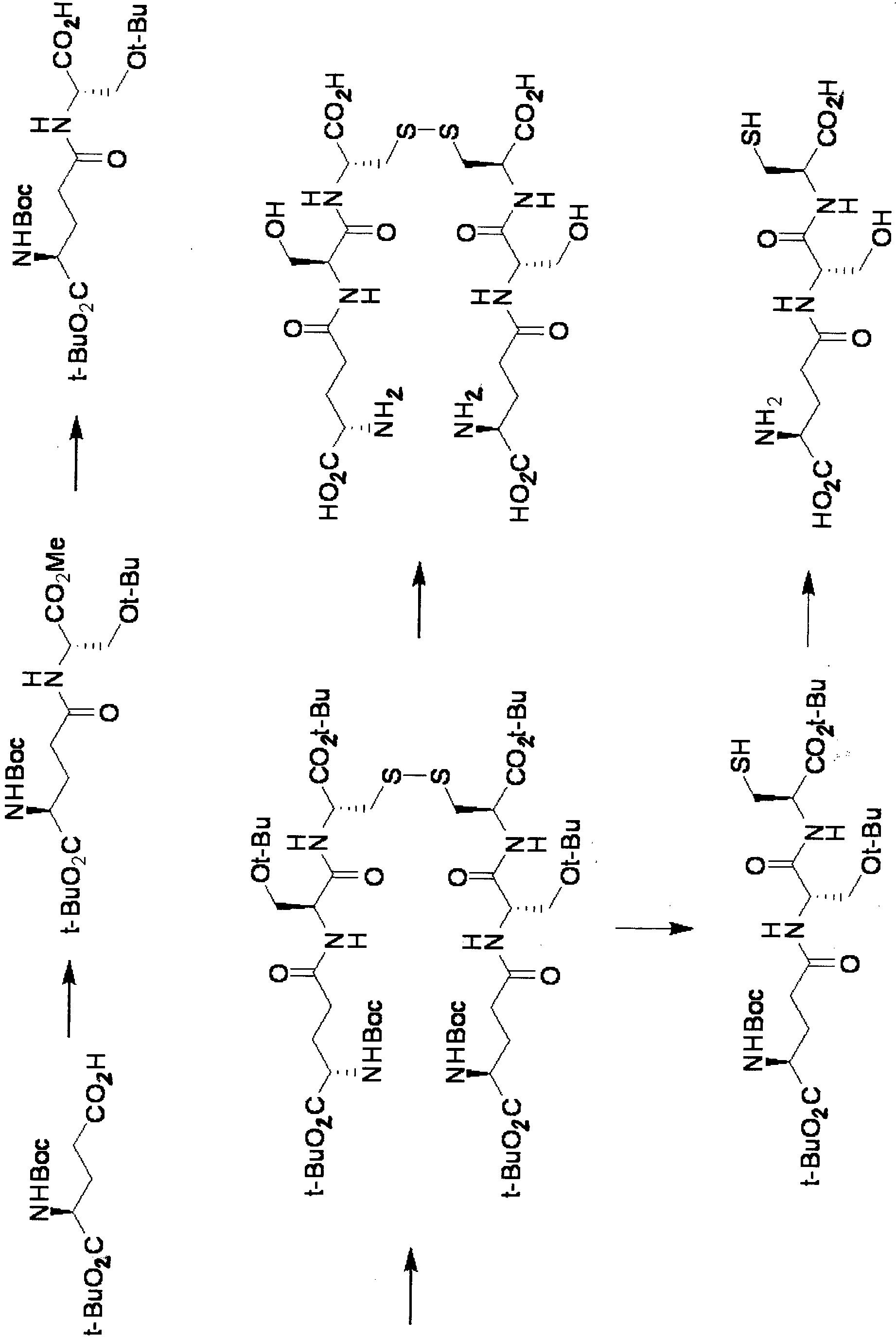 Compound (I) in which X is an optionally substituted oxygen atom or a halogen atom and Y is an optionally substituted sulfur atom can be synthesized according to the following synthetic scheme III. 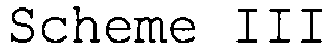 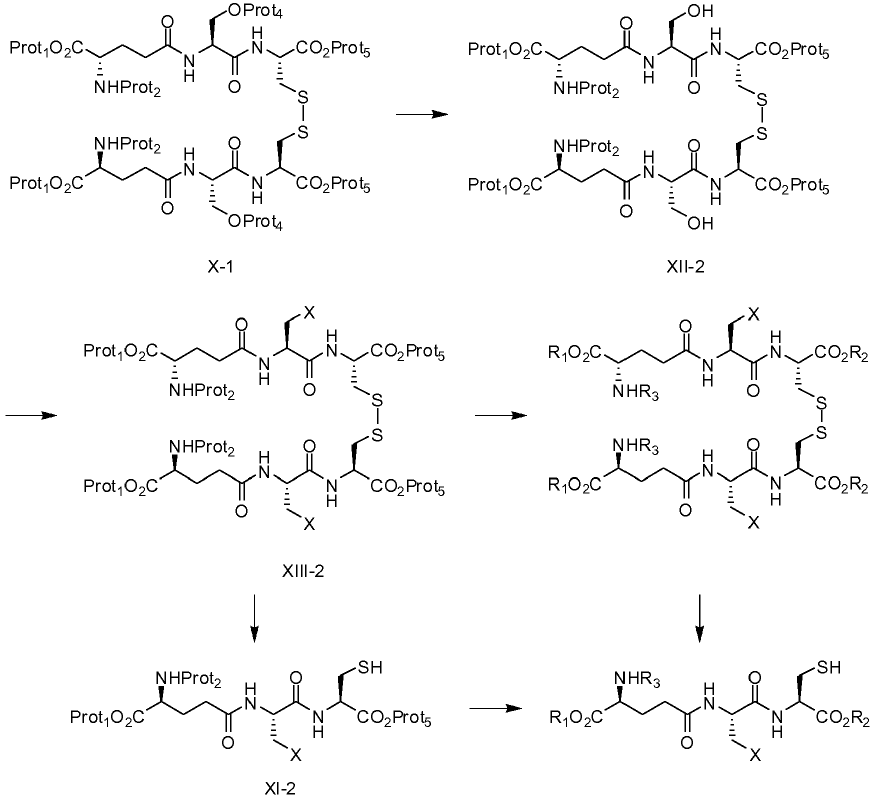
wherein Prot1 to Prot5 each independently represents a suitable protective group.

Specifically, compound (XII-2) obtained by removing the protective group 4 (Prot4) of compound (X-1) obtained in scheme I can be converted to a phosphoric acid derivative or a sulfonic acid derivative by using a commonly used reagent, for example, phosphonyl chloride or sulfonyl chloride or to a halogen derivative by using a halogen with a phosphine derivative or the like. Direct deprotection of the obtained (XIII-2) makes it possible to obtain oxidatively dimerized compound (I). On the other hand, thiol compound (XI-2) can be obtained by reducing the disulfide bond of compound (XIII-2) with, for example, a phosphine derivative, and reduced compound (I) can be obtained by removing the protective groups of compound (XI-2). Reduced compound (I) can also be obtained by reducing oxidized compound (I) with a phosphine derivative as in the above-described case. The thus obtained compounds represented by general formula (I) can be isolated and purified by known separation **techniques,** such as **concentration under reduced pressure,** solvent extraction, crystallization, and chromatography.

Compound (I) in which X is an optionally substituted oxygen atom or a halogen atom and Y is an optionally substituted sulfur atom can be synthesized also according to the following synthetic scheme IV. 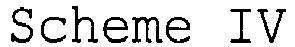 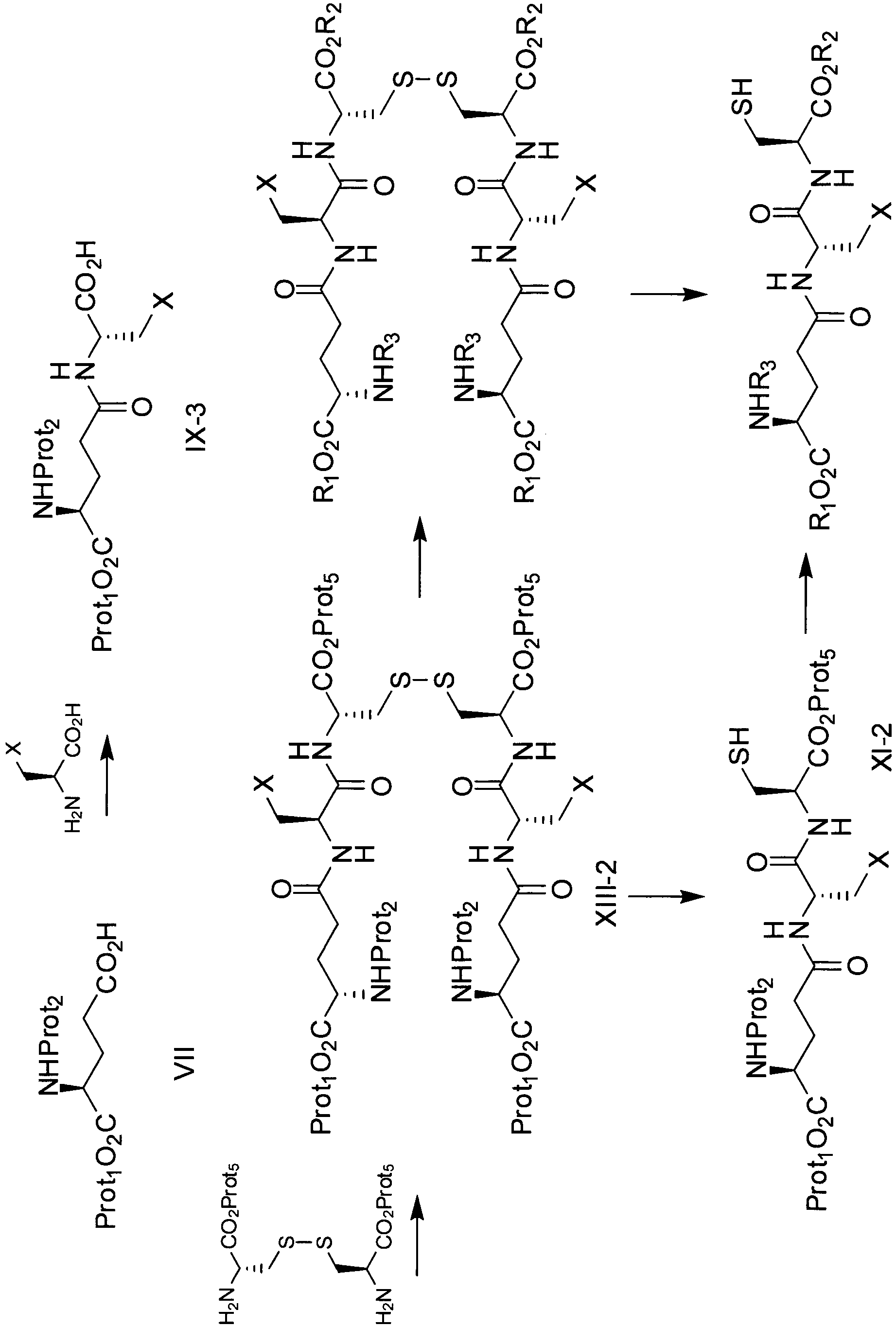 Specifically, first, dipeptide (IX-3) is obtained from glutamic acid derivative (VII) and a substituted alanine derivative by using a commonly used condensation agent. Next, compound (IX-3) and a cystine derivative are condensed to prepare tripeptide (XIII-2). Direct deprotection of tripeptide (XIII-2) makes it possible to obtain oxidatively dimerized compound (I). On the other hand, thiol compound (XI-2) can be obtained by reducing the disulfide bond of compound (XIII-2), for example, with a phosphine derivative, and reduced compound (I) can be obtained by removing protective groups of compound (XI-2). The reduced compound can also be obtained by reducing oxidized compound (I) with a phosphine derivative as in the above-described case. The thus obtained compounds represented by general formula (I) can be isolated and purified by known separation **techniques**, such as **concentration under reduced pressure,** solvent extraction, crystallization, and chromatography.

Compound (I) in which X is an optionally substituted sulfur atom and Y is an optionally substituted oxygen atom or a halogen atom can be synthesized according to the following synthetic scheme V. 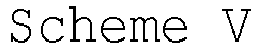 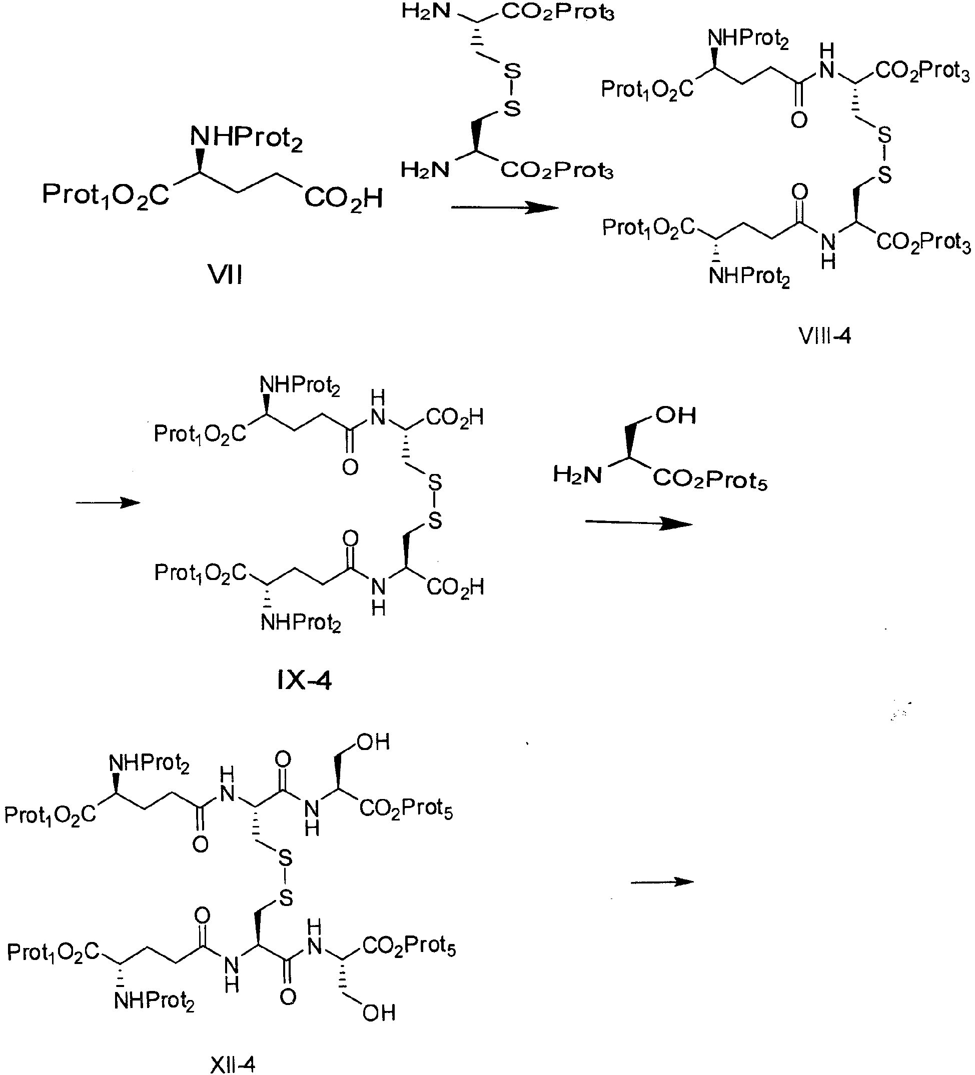 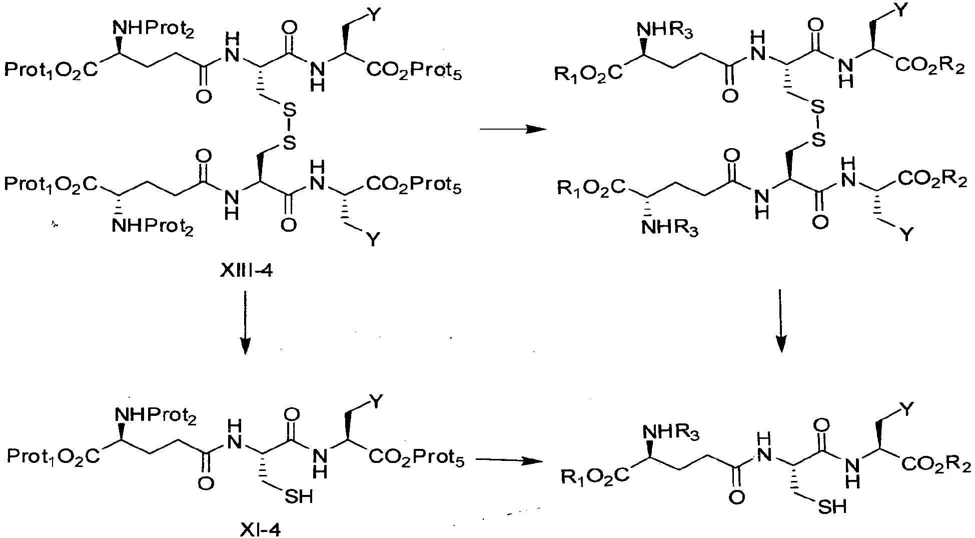 wherein Prot1 to Prot5 each independently represents a suitable protective group.

Specifically, first, dipeptide (VIII-4) is obtained from glutamic acid derivative (VII) and a cystine derivative by using a commonly used condensation agent. Next, carboxylic acid (IX-4) obtained by hydrolysis of compound (VIII-4) and a serine derivative are condensed to prepare tripeptide (XII-4). Tripeptide (XII-4) can be converted to a phosphoric acid derivative or a sulfonic acid derivative with a commonly used reagent, for example, phosphonyl chloride or sulfonyl chloride, or to a halogen derivative by using a halogen with a phosphine derivative or the like. Direct deprotection of the obtained (XIII-4) makes it possible to obtain oxidatively dimerized compound (I). On the other hand, thiol compound (XI-4) can be obtained by reducing the disulfide bond of compound (XIII-4), for example, with a phosphine derivative, and reduced compound (I) can be obtained by removing the protective groups of compound (XI-4). The reduced compound can also be obtained by reducing oxidized compound (I) with a phosphine derivative as in the above-described case. The thus obtained compounds represented by general formula (I) can be isolated and purified by known separation **techniques**, such as **concentration under reduced pressure**, solvent extraction, crystallization, and chromatography.

Compound (I) in which X is an optionally substituted sulfur atom and Y is an optionally substituted oxygen atom or a halogen atom can also be synthesized according to the following synthetic scheme VI. 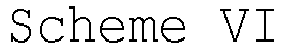 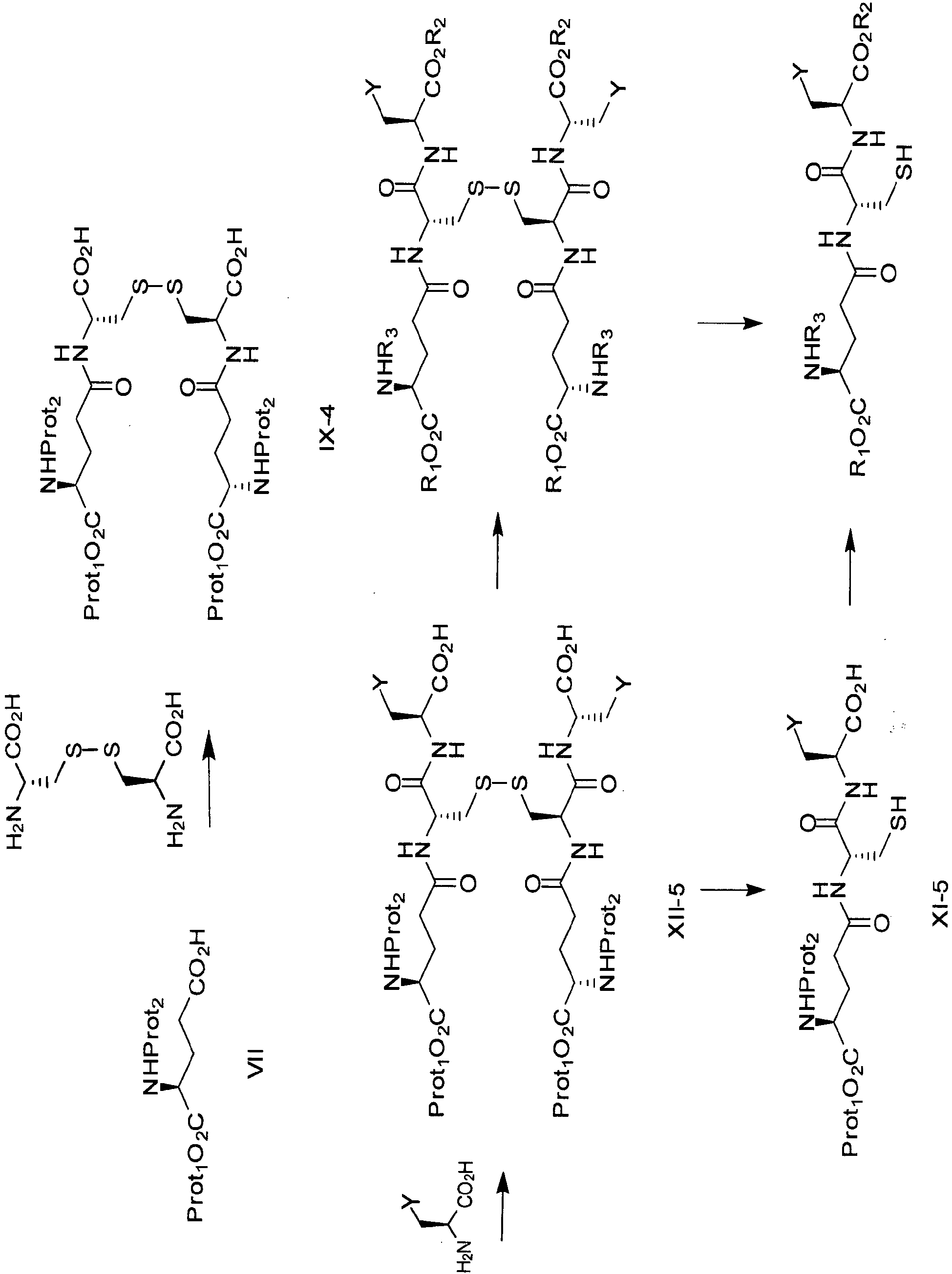 Specifically, first, dipeptide (IX-4) is obtained from glutamic acid derivative (VII) and cystine by using a commonly used condensation agent. Next, compound (IX-4) and a substituted alanine derivative are condensed to prepare tripeptide (XII-5). Direct deprotection of tripeptide (XII-5) makes it possible to obtain oxidatively dimerized compound (I). On the other hand, thiol compound (XI-5) can be obtained by reducing the disulfide bond of compound (XII-5), for example, with a phosphine derivative, and reduced compound (I) can be obtained by removing the protective groups of compound (XI-5). The reduced compound can also be obtained by reducing oxidized compound (I) with a phosphine derivative as in the above-described case. The thus obtained compounds represented by general formula (I) can be isolated and purified by known separation **techniques**, such as **concentration under reduced pressure,** solvent extraction, crystallization, and chromatography.

The compound represented by formula (V) or a chemically acceptable salt thereof of the present invention is preferably any one of the following compounds or a chemically acceptable salt thereof. 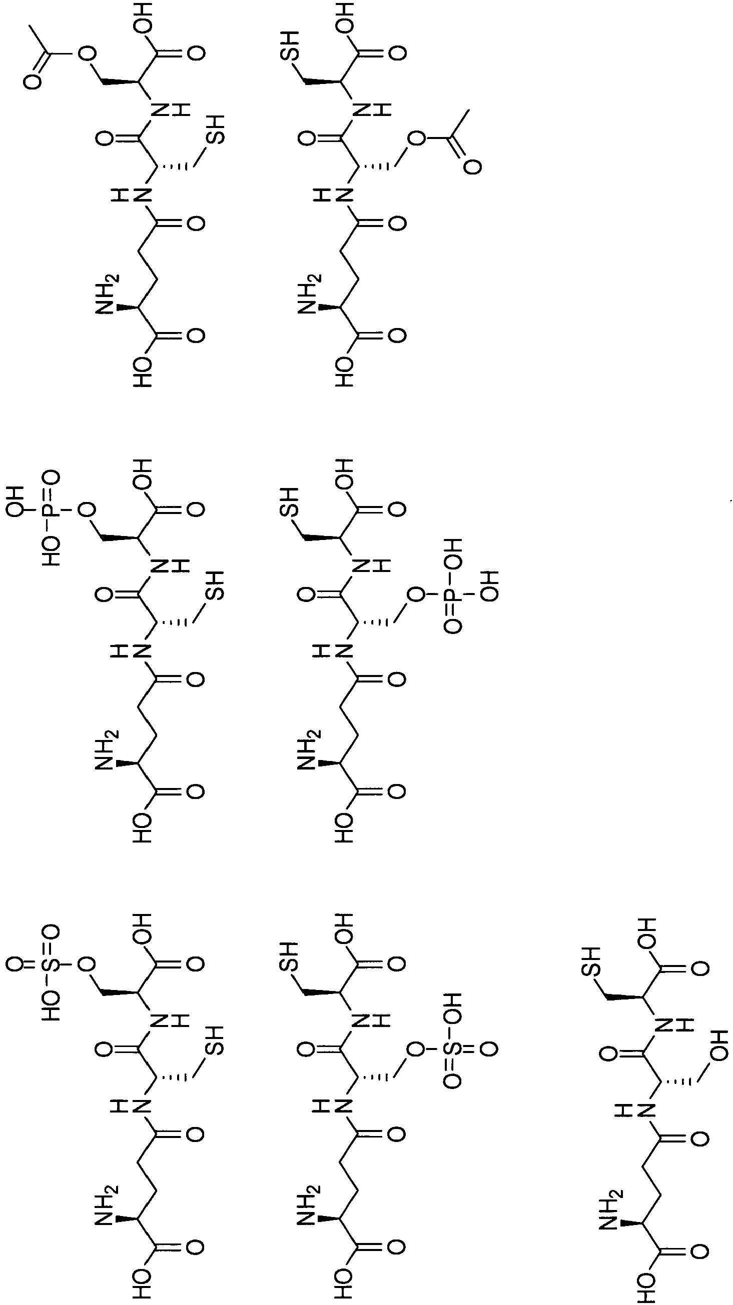 In addition, the compound represented by formula (V) or a chemically acceptable salt thereof of the present invention is preferably any one of γ-Glu-Ser-Cys, γ-Glu-Ser(SO₃H)-Cys, γ-Glu-Ser(PO₃H₂)-Cys, γ-Glu-Ser(Ac)-Cys, γ-Glu-Cys-Ser(SO₃H), γ-Glu-Cys-Ser(PO₃H₂), and γ-Glu-Cys-Ser(Ac). Here, Ser(SO₃H) represents serine sulfuric acid ester, Ser(PO₃H₂) represents phosphoserine, and Ser(Ac) represents O-acetylserine.

Moreover, the compound represented by formula (V) of the present invention also includes, for example, compounds (oxidative dimers) in which two molecules of a compound(s) represented by formula (V) in which X' or Y' is SH are dimerized via a disulfide bond. An example of the oxidative dimer is the compound shown below.

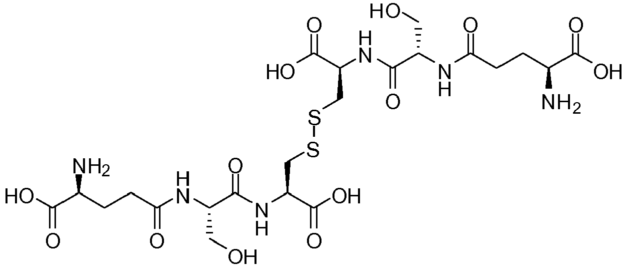

The compound obtained by the production method of the present invention and represented by formula (II) or (III) can be isolated and purified by known separation **techniques** such as **concentration under reduced pressure**, solvent extraction, crystallization, and chromatography.

The lanthionine derivative produced by the production method of the present invention has an excellent CaSR agonist activity and an excellent kokumi-imparting effect, and hence can be used as a CaSR agonist and a kokumi-imparting agent. Accordingly, the compound represented by formula (V) or a chemically acceptable salt thereof, which is an intermediate compound, can be used for production of a CaSR agonist, a kokumi-imparting agent, or a food and/or beverage ingredient. When the compound represented by formula (V) or a chemically acceptable salt thereof, which is an intermediate compound, is used for production of a CaSR agonist, a pharmaceutically acceptable carrier, diluent, or the like may be added to the CaSR agonist, if necessary. In addition, the compound represented by formula (V) or a chemically acceptable salt thereof can be added in such an amount that the amount of the lanthionine derivative contained can be 10 ppb to 99.9%, preferably 0.05 ppm to 99.9%, and more preferably 0.1 ppm to 99.9%, relative to the weight of the CaSR agonist. Likewise, when the compound represented by formula (V) or a chemically acceptable salt thereof, which is an intermediate compound, is used for production of a kokumi-imparting agent or a food and/or beverage ingredient, various edible food additives and the like may be added to the kokumi-imparting agent or the food and/or beverage ingredient. In addition, the compound represented by formula (V) or a chemically acceptable salt thereof can be added in such an amount that the amount of the lanthionine derivative contained can be 10 ppb to 99.9%, preferably 0.05 ppm to 99.9%, and more preferably 0.1 ppm to 99.9%, relative to the weight of the kokumi-imparting agent or the food and/or beverage ingredient.

In addition, the use of the lanthionine derivative produced by the production method of the present invention in combination with at least one other ingredient for seasoning selected from amino acids such as sodium glutamate (MSG), nucleic acids such as inosine monophosphate (IMP), inorganic salts such as sodium chloride, organic acids such as citric acid, various yeast extracts, and the like makes it possible to provide a favorable seasoning composition having a more enhanced kokumi than that achieved by the use of the other ingredient for seasoning by itself. When the lanthionine derivative produced by the production method of the present invention is used in combination with the above-described other ingredient for seasoning, those skilled in the art can set as appropriate the concentration of the lanthionine derivative based on a test such as sensory evaluation. For example, the lanthionine derivative of the present invention may be contained so that the final concentration thereof can be approximately 0.1 ppm to 500 ppm.

In the present invention, the "kokumi" means a taste which cannot be expressed by the five basic tastes consisting of the sweet taste, the salty taste, the sour taste, the bitter taste, and the umami taste (savoriness), and refers to a taste in which not only a basic taste(s), but also a marginal taste (s) of the basic taste(s), such as the thickness, the growth (mouthfulness), the continuity, and the harmony is(are) enhanced. Here, "kokumi impartment" refers to enhancement of any of the five basic tastes represented by the sweet taste, the salty taste, the sour taste, the bitter taste, and the umami taste, and accompanying impartment of any of the marginal tastes of the basic tastes such as the thickness, the growth, and the harmony. Moreover, this "kokumi impartment" can also be expressed as a flavor enhancement effect. Accordingly, the compound produced by the production method of the present invention can also be expressed as a flavor enhancer. The compound produced by the production method of the present invention can also be used as a sweet-taste enhancer, a salty-taste enhancer, a sour-taste enhancer, a bitter-taste enhancer, or an umami-taste enhancer.

In addition, the taste of a food changes with time after the food is taken into the mouth, and the changing tastes are referred to as the initial taste, the middle taste, and the after taste in this order from the time immediately after the food is taken into the mouth. These are concepts determined relative to each other. In general, the initial taste, the middle taste, and the after taste are respectively a taste perceived from 0 to 2 seconds, a taste perceived from 2 seconds to 5 seconds, and a taste perceived on and after 5 seconds, after the food is taken into the mouth. In addition, the initial taste and the middle taste are collectively referred to as the "initial-middle taste," while the middle taste and the after taste are collectively referred to as the "middle-after taste." In other words, the taste perceived from 0 to 5 seconds is referred to as the "initial-middle taste," and the taste perceived from 2 seconds to around 30 seconds is referred to as the "middle-after taste." If the taste is evaluated with the three divided periods, it is difficult for a person who takes the food to concentrate his/her attention on the evaluation. Hence, evaluation with the two divided periods is usually employed.

The effect of a substance possessing a CaSR activity on the kokumi and a taste pattern can be checked by methods such as a taste test conducted by the human. Examples of such a sensory taste test conducted by the human include the test shown in Example 13 of Patent Literature 2 (Japanese Patent Application Publication No. 2011-115186), but are not limited thereto.

The "CaSR" herein means the calcium sensing receptor, which belongs to class C of the seven transmembrane receptors, and is also called the calcium receptor. The "CaSR agonist" herein means a substance which binds to the CaSR and activates the CaSR. Moreover, the "activation of the CaSR" herein means that a ligand binds to the CaSR, activates the guanine nucleotide-binding protein, and causes signals to be transmitted. In addition, the property of binding to the CaSR and activating the CaSR is referred to as the "CaSR agonist activity."

A preferred example of the CaSR is the human CaSR coded by the human CaSR gene registered under GenBank Accession No. NM_000388. Note that the CaSR is not limited to the protein coded by the gene having the sequence. As long as a protein having a CaSR function is coded, the protein may be coded by a gene having a homology not less than 60%, preferably not less than 80%, and more preferably not less than 90% to the above-described sequence. Note that the CaSR function can be investigated by expressing the gene in a cell, and measuring the change of a current or the change in concentration of the intracellular calcium ions upon addition of calcium.

The origin of the CaSR is not particularly limited, and the CaSR may be not only the above-described human CaSR, but also a CaSR derived from any one of animals including mice, rats, dogs and the like.

As described above, the CaSR activity can be checked by using living cells expressing the CaSR or a fragment thereof, a cell membrane expressing the CaSR or a fragment thereof, an in vitro system containing the CaSR or a protein of a fragment thereof, or the like.

One example in which living cells are used is shown below, but the present invention is not limited thereto.

The CaSR is expressed in cultured cells such as Xenopus laevis oocytes, hamster ovary cells or human embryonic kidney cells. This is made possible by introducing a material obtained by cleaning a CaSR gene in a plasmid carrying an exogenous gene in a state of the plasmid, or by introducing a cRNA obtained by using this material as a template. The reaction can be detected by using an electrophysiological approach or a fluorescent indicator which indicates the rise in intracellular calcium.

The expression of the CaSR is first checked based on the response to calcium or a specific activator. Oocytes in which an intracellular current is observed in response to calcium in a concentration of about 5 mM or cultured cells in which fluorescence of a fluorescent indicator is observed in response thereto are used. The concentration dependence is measured by changing the calcium concentration. Next, a test substance is prepared in a concentration of about 1 µM to 1 mM, and added to the oocytes or the cultured cells. Then, the CaSR activity is measured in the presence of the test substance to determine the CaSR agonist activity of the test substance.

More specific examples of the test for the CaSR agonist activity include the tests shown in Examples described in International Publication No. W02007/055393, but the test for the CaSR agonist activity is not limited thereto.

Regarding amino acids or peptides used in combination with the lanthionine derivative produced by the production method of the present invention, peptides described in International Publication No. W02007/055393 may be used in combination.

The lanthionine derivative produced by the production method of the present invention can be used as a seasoning composition by itself or after being mixed with a carrier acceptable for foods and beverages or other ingredients for seasoning. Examples of the other ingredients for seasoning include flavors; saccharides; sweeteners; dietary fibers; vitamins; amino acids such as sodium glutamate (MSG); nucleic acids such as inosine monophosphate (IMP); inorganic salts such as sodium chloride; and organic acids such as citric acid; as well as various yeast extracts.

The lanthionine derivative produced by the production method of the present invention includes those in the forms of salts. When the lanthionine derivative produced by the production method of the present invention can form a salt, the salt thereof only needs to be a chemically and pharmacologically acceptable and edible salt. Examples of the salt include ammonium salt; salts with alkali metals such as sodium and potassium; salts with alkaline earth metals such as calcium and magnesium; aluminum salt; zinc salt; salts with organic amines such as triethylamine, ethanolamine, morpholine, pyrrolidine, piperidine, piperazine, and dicyclohexylamine; salts with basic amino acids such as arginine and lysine, in the case of acidic groups such as a carboxyl group. Meanwhile, in the case of basic groups, examples of the salt include salts with inorganic acids such as hydrochloric acid, sulfuric acid, phosphoric acid, nitric acid, and hydrobromic acid; salts with organic carboxylic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, teoclic acid, salicylic acid, lactic acid, oxalic acid, mandelic acid, and malic acid; salts with organic sulfonic acids such as methanesulfonic acid, benzenesulfonic acid, and p-toluenesulfonic acid.

Each of the lanthionine derivative produced by the production method of the present invention, the CaSR agonist, the kokumi-imparting agent, and the food and/or beverage ingredient can be used in any form such as a dry powder, a paste, or a solution without any limitation on the physical properties thereof.

Each of the lanthionine derivative produced by the production method of the present invention, the CaSR agonist, the kokumi-imparting agent, and the food and/or beverage ingredient can be used by being blended with various foods and beverages such as foods, beverages, and seasoning compositions.

When each of the lanthionine derivative produced by the production method of the present invention, the CaSR agonist, the kokumi-imparting agent, and the food and/or beverage ingredient is used by being blended with various foods and beverages such as foods, beverages, and seasoning compositions, the final amount of the lanthionine derivative and the final amount of the amino acid(s) and/or peptide(s) used in combination are not particularly limited, as long as a desired effect can be obtained. The amount of the lanthionine derivative and the amount of the amino acid(s) or peptide(s) are each about 10 ppb to 99.9%, preferably 0.05 ppm to 99.9%, and more preferably 0.1 ppm to 99.9%, based on the total mass of the food, the beverage, the seasoning composition, or the like.

Various foods and beverages such as foods, beverages, and seasoning compositions blended with the lanthionine derivative produced by the production method of the present invention, the CaSR agonist, the kokumi-imparting agent, or the food and/or beverage ingredient may further be blended with any solid or liquid carrier acceptable for foods and beverages, a suitable ingredient for seasoning, or the like.

Examples of the carrier include glucose, lactose, sucrose, starch, mannitol, dextrin, fatty acid glycerides, polyethylene glycol, hydroxyethyl starch, ethylene glycol, polyoxyethylene sorbitan fatty acid esters, gelatin, albumin, amino acids, water, saline, and the like.

The above-described ingredient for seasoning may be any ingredient for seasoning used in this technical field, and is not particularly limited. More specific examples thereof include those described earlier.

The content of each of the carrier, the other ingredient for seasoning, and the like is not particularly limited.

Of the ingredients for seasoning described earlier, the yeast extracts are not particularly limited in terms of fungal cells from which the yeast extracts are derived, of the conditions for culturing the fungal cells, and of the treatment method for extraction from the fungal cells, and any yeast extracts can be used. The yeast extracts may further be subjected to a heat treatment, an enzyme treatment, concentration, a granulating treatment, or the like.

The present invention is described more specifically based on examples shown below. However, the present invention is not limited to these examples.

### Examples

### (Reference Example) Production of compound represented by formula (III) (compound represented by formula (II) where R₁, R₂, and R₃ are all hydrogen)

(Fmoc-L-Cys-Ot-Bu)₂ (N,N'-difluorenylmethoxycarbonyl-L-cystine di-t-butyl ester, 4.81 mmol) was dissolved in tetrahydrofuran (58.5 mL) and water (1.5 mL). Under ice-cooling, tributylphosphine (5.28 mmol) was added thereto, and the temperature was returned to room temperature, followed by stirring for 4 hours. The reaction liquid was cooled, and a 10% aqueous citric acid solution (60 mL) was added. The temperature of the white turbid liquid was returned to room temperature, followed by extraction with ethyl acetate (60 mL). The organic layer was washed with 60 mL of aqueous sodium chloride, and then concentrated to obtain an oily residue. The residue was purified with a silica gel column (n-hexane-ethyl acetate) to obtain compound 1 as an oily substance.
Yield: 97%.
ESI MS m/z 422.4 (M+Na)⁺
¹H NMR (400 MHz, CDCl₃) δ 1.50 (9H, s), 2.99 (2H, m), 4.23 (1H, t, J=6. Hz), 4.41 (2H, m), 4.54 (1H, m), 5.68 (1H, d, J=7.2 Hz), 7.32 (2H, m), 7.41 (2H, t, J=7.2 Hz), 7.61 (2H, d, J=7.6 Hz), 7.77 (2H, d, J=7.2 Hz).

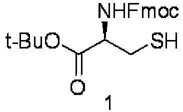

### Synthesis of compound 2

Compound 1 (6.04 mmol) was dissolved in anhydrous dimethylformamide (60 mL), and Boc-iodo-D-Ala-OMe (N-t-butoxycarbonyl-3-iodo-D-alanine methyl ester) (6.20 mmol) was added. Then, cesium carbonate (6.02 mmol) was added thereto, followed by stirring at room temperature overnight. The reaction liquid was cooled, and a 10% aqueous citric acid solution (50 mL) and water (30 mL) were added thereto, followed by extraction with ethyl acetate (60 mL). Then, the aqueous layer was again extracted with ethyl acetate (60 mL). The organic layers were combined, washed sequentially with a 10% aqueous citric acid solution (50 mL) and an aqueous sodium chloride (50 mL), and then concentrated. The obtained oily residue was purified with a silica gel column (n-hexane-ethyl acetate) to obtain compound 2 as an oily substance.
Yield: 66%.
ESI MS m/z 601.2 (M+H)⁺
¹H NMR (400 MHz, CDCl₃) δ 1.45 (9H, s), 1.49 (9H, s), 3.01 (4H, m), 3.73 (3H, s), 4.24 (1H, t, J=7.2 Hz), 4.39 (2H, d, J=7.2 Hz), 4.48-4.55 (2H, m), 5.37 (1H, brd, J=6.8 Hz), 5.80 (1H, brd, J=6.8 Hz), 7.32 (2H, m), 7.40 (2H, t, J=7.2 Hz), 7.63 (2H, m), 7.77 (2H, d, J=7.6 Hz).

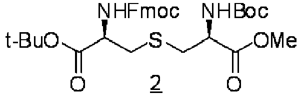

### Synthesis of compound 4

(Step 1) Compound 2 (4.01 mmol) was dissolved in 70 mL of dichloromethane, and trifluoroacetic acid (70 mL) was added thereto, followed by stirring at room temperature for 1 hour. Then, the reaction liquid was concentrated to obtain a residue containing compound 3. The yield was assumed to be 100%, and the residue was used as it was in the subsequent reaction.

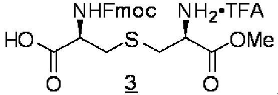

(Step 2) Under ice-cooling, anhydrous dimethylformamide (60 mL) was added to compound 3 (equivalent to 4.01 mmol) to obtain a uniform liquid. To this liquid, diisopropylethylamine (8.04 mmol) was added dropwise. The temperature was returned to room temperature, and carbonylbisimidazole (8.10 mmol) was added thereto, followed by stirring overnight as it was. Under a cooling and stirring condition, a 10% aqueous citric acid solution (50 mL) was added to the reaction liquid, and the temperature was returned to room temperature, followed by extraction with ethyl acetate (100 mL). The aqueous layer was further extracted with ethyl acetate, and the organic layers were combined. The combined organic layer was washed with a 10% aqueous citric acid solution (50 mL x 2) and once with aqueous sodium chloride, and then concentrated to obtain an oily residue. The obtained residue was purified with a silica gel column (n-hexane-ethyl acetate) to obtain compound 4 as an oily substance.
Yield: 39% (two steps).
ESI MS m/z 448.5 (M+Na)⁺
¹H NMR (400 MHz, CDCl₃) δ 2.71 (1H, dd, J=9.2, 14.4 Hz), 2.78-2.90 (2H, m), 3.02 (1H, d, J=14.4 Hz), 3.86 (3H, s), 4.20 (1H, t, J=6. Hz), 4.40 (2H, d, J=6.8 Hz), 4.56 (1H, dd, J=5.6, 9.2 Hz), 4.68 (1H, m), 6.30 (1H, d, J=5.6 Hz), 7.32 (2H, t, J=7.6 Hz), 7.40 (2H, t, J=7.6 Hz), 7.60 (2H, d, J=7.6 Hz), 7.77 (2H, d, J=7.6 Hz).

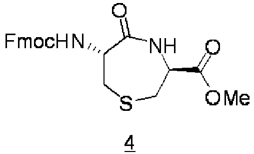

### Synthesis of compound 6

(Step 1) To compound 4 (1.54 mmol), a 10% morpholine-dimethylformamide solution (14 mL) was added, followed by stirring at room temperature for 30 minutes. The reaction liquid was concentrated to obtain a residue containing compound 5. The yield was assumed to be 100%, and the residue was used in the subsequent reaction as it was.

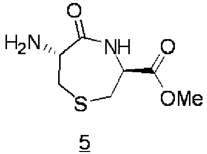

(Step 2) Boc-Glu-OtBu (N-t-butoxycarbonyl-L-glutamic acid α-t-butyl ester) (1.70 mmol) was dissolved in anhydrous dimethylformamide (9 mL), and HOBt·H₂O (1-hydroxybenzotriazole hydrate) (1.85 mmol) a n d WSC·HCl (1-(3-dimethylaminopropyl)-3-ethylcarbodiimide hydrochloride) (1.90 mmol) were added thereto, followed by stirring at room temperature for 15 minutes. To this mixture, compound 5 (equivalent to 1.54 mmol) suspended in dimethylformamide (20 mL) was added, and the reaction was allowed to proceed at room temperature overnight. The reaction liquid was concentrated, followed by phase separation by adding ethyl acetate (50 mL) and water (50 mL) to the residue. The aqueous layer was further extracted with ethyl acetate (50 mL) . The organic layers were combined, washed with aqueous sodium hydrogen carbonate (50 mL) and with aqueous sodium chloride (50 mL), and then concentrated. The obtained paste-like residue was purified with a silica gel column (n-hexane-ethyl acetate) to obtain 6 as an oily substance.
Yield: 81% (two steps).
ESI MS m/z 490.0 (M+H)⁺
¹H NMR (300 MHz, CDCl₃) δ 1.44 (9H, s), 1.46 (9H, s), 1.90 (1H, m), 2.17 (1H, m), 2.32 (2H, m), 2.60 (1H, dd, J=10.5, 14.1 Hz), 2.92-2.98 (2H, m), 3.18 (1H, dd, J=6.0, 14.7 Hz), 3.84 (3H, s), 4.46 (1H, m), 4.80 (1H, m), 5.19 (1H, d, J=8.1 Hz), 6.32 (1H, d, J=8.1 Hz), 7.09 (1H, brs).

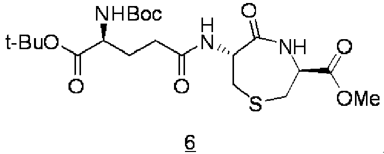

### Synthesis of compounds 8a and 8b

(Step 1) Compound 6 (1.25 mmol) was dissolved in tetrahydrofuran (30 mL), and a 0.2 M aqueous lithium hydroxide solution (2.50 mmol) was added with stirring under ice-cooling. Thirty minutes later, the liquid was neutralized to a pH of approximately 6 with a 10% aqueous citric acid solution. The temperature was returned to room temperature, and the reaction liquid was concentrated, followed by extraction with ethyl acetate (20 mL x 3). The aqueous layer was further extracted with ethyl acetate (20 mL x 3). The organic layers were combined, and washed with aqueous sodium chloride (10 mL). Then, the organic layer was concentrated to obtain compound 7. The yield was assumed to be 100%, and the compound 7 was used in the subsequent reaction as it was.

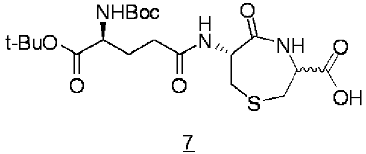

(Step 2) To compound 7 (equivalent to 1.25 mmol), a 4 N HCl/dioxane solution (25 mL) was added, and the reaction was allowed to proceed at room temperature overnight. Then, the reaction liquid was concentrated. The obtained paste-like residue was purified with a strongly basic ion-exchange resin (Amberlite IRA400 OH AG) to obtain two fractions. A portion of the first eluted fraction was further purified by reverse-phase preparative HPLC (column: Develosil RPAQUEOUS-AR-5 manufactured by Nomura Chemical Co., Ltd, mobile phase: linear gradient of water/acetonitrile containing 0.1% formic acid) to obtain compound 8a as a sticky substance (candy-like substance). On the other hand, the second eluted fraction was concentrated to obtain a white solid, and the solid was dissolved in water and freeze dried. The obtained residue was slurry-washed with a small amount of water to obtain compound 8b as a white solid.

### Compound 8a

ESI MS m/z 318.3 (M-H)⁻
¹H NMR (600 MHz, D₂O) δ 2.13 (2H, m), 2.50 (2H, t, J=7.8 Hz), 2.64 (1H, d, J=15.0 Hz), 2.83 (1H, dd, J=10.8, 15.0 Hz), 3.02 (1H, dd, J=2.4, 15.0 Hz), 3.15 (1H, dd, J=5.4, 15.0 Hz), 3.83 (1H, t, J=6.0 Hz), 4.55 (1H, dd, J=2.4, 5.4 Hz), 4.91 (1H, dd, J=2.4, 10.8 Hz).

### Compound 8b

Yield: 26% (two steps)
ESI MS m/z 318.0 (M-H)⁻
¹H NMR (600 MHz, D₂O) δ 2.12 (2H, m), 2.48 (2H, t, J=7.2 Hz), 2.72 (1H, d, J=14.4 Hz), 2.76 (1H, dd, J=10.2, 14.4 Hz), 2.89 (1H, dd, J=9.6, 14.4 Hz), 3.05 (1H, d, J=14.4 Hz), 3.78 (1H, t, J=6.0 Hz), 4.42 (1H, d, J=9.6 Hz), 4.91 (1H, d, J=10.2 Hz).

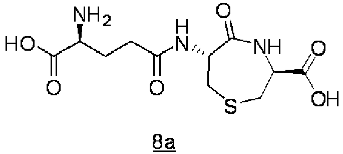

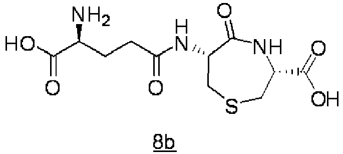

### Example 1: Synthesis (1) of compound represented by formula (III) (compound represented by formula (II) where R₁, R₂, and R₃ are all hydrogen) using compound represented by formula VI

γ-Glu-Cys-Cys(VI) synthesized with reference to Non Patent Literature 2 was dissolved in ultra pure water to prepare a pH 3.3 aqueous sample solution of a concentration of 53 ppm. A portion of the pH 3.3 aqueous sample solution was taken out, and the pH was adjusted to 10 by adding an aqueous sodium hydroxide solution. Then, 100 µL of the solution was dispensed to a 1.5 mL micro test tube. The dispensed aqueous solution was heated for 6 hours with a water bath at 90°C.

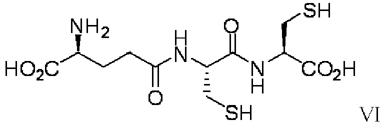

The obtained heat-treated sample was subjected to separation by reverse-phase liquid chromatography shown below, and introduced into a mass spectrometer to quantify the amount of the compound represented by formula (III) contained in the sample. As a standard for the quantification, the compound represented by formula (III) and synthesized by the method of Reference Example above was used.

Separation conditions of the following reverse-phase liquid chromatography were as follows.
(1) HPLC: Agilent Technologies 1200 series
(2) Separation column: Develosil RPAQUEOUS, inner diameter: 2.0 mm, length: 250 mm, (manufactured by Nomura Chemical Co., Ltd)
(3) Injection volume: 2 µL
(4) Column temperature: 25°C
(5) Mobile phase A: 0.1% acetic acid/acetonitrile = 98/2
(6) Mobile phase B: 0.1% acetic acid/acetonitrile = 10/90
(7) Flow rate: 0.2 mL/min
(8) Elution conditions: The component was eluted by using a mixture liquid of mobile phase A and mobile phase B. The ratio of mobile phase B in the mixture liquid was as follows: 0 minutes (0%), 20 minutes (60%), 20.1 minutes (0%), 30 minutes (0%)

Quantification conditions with a mass spectrometer were as follows.
(1) Mass spectrometer: Applied Biosystems API4000
(2) Detection mode: Selected Ion Monitoring (positive ion mode)
(3) Selected ions:

| | First mass analyzer | Second mass analyzer |
|---|---|---|
| | Q1 | Q3 |
| Compound III | 320.2 | 190.9 |

(4) Collision energy (CE): 19 (V)
(5) Quantification method: Quantification was conducted by the external standard method using the sample of the compound represented by formula (III) (the compound synthesized in Reference Example above).

### Example 2: Synthesis (2) of compound represented by formula (III) (compound represented by formula (II) where R₁, R₂, and R₃ are all hydrogen) using compound represented by formula VI

The pH 3.3 aqueous sample solution (8 mL) prepared in Example 1 was adjusted to pH 12 by adding an aqueous sodium hydroxide solution, and then 100 µL of the solution was dispensed to a 1.5 mL micro test tube. Then, the sample heat-treated for 6 hours was prepared by the same method as in Example 1, and then the amount of Compound III was quantified.
Yield

The reaction yields were as follows.

**Table 1**

| | Yield (%) |
|---|---|
| Example 1 | 10.4 |
| Example 2 | 13.0 |

### Example 3: Synthesis (3) of compound represented by formula (III) (compound represented by formula (II) where R₁, R₂, and R₃ are all hydrogen) using compound represented by formula VI

γ-Glu-Cys-Cys(VI) was dissolved in ultra pure water to prepare a pH 3.3 aqueous sample solution of a concentration of 0.3 mM (106 ppm). To the pH 3.3 aqueous sample solution (0.5 mL), a 200 ppm aqueous iron(III) nitrate solution (0.5 mL) was added, and then the pH was adjusted to 10 with an aqueous sodium hydroxide solution. This aqueous solution was further heated for 5 hours with a water bath at 90°C. The amount of Compound III in this heat-treated sample was quantified.

### Example 4: Synthesis (4) of compound represented by formula (III) (compound represented by formula (II) where R₁, R₂, and R₃ are all hydrogen) using compound represented by formula VI

To the pH 3.3 aqueous sample solution (0.5 mL) prepared in Example 3, a 400 ppm aqueous calcium carbonate solution (0.5 mL) was added, and then the pH was adjusted to 10 with an aqueous sodium hydroxide solution. This aqueous solution was further heated for 3 hours with a water bath at 90°C. The amount of Compound III in the heat-treated sample was quantified.

### Example 5: Synthesis (5) of compound represented by formula (III) (compound represented by formula (II) where R₁, R₂, and R₃ are all hydrogen) using compound represented by formula VI

γ-Glu-Cys-Cys(VI) was dissolved in ultra pure water to prepare a pH 3.3 aqueous sample solution of a concentration of 106 ppm. To the pH 3.3 aqueous sample solution (0.5 mL), a 10 ppm aqueous zinc nitrate solution (0.5 mL) was added, and then the pH was adjusted to 10 with an aqueous sodium hydroxide solution. This solution was heated for 6 hours with a water bath at 90°C. The amount of Compound III in this heat-treated sample was quantified.

The results of improvement in the yield of Compound III achieved by adding metal salts were as follows.

**Table 2**

| | Compound III yield improvement effect (%) |
|---|---|
| Example 3 | 139 |
| Example 4 | 131 |
| Example 5 | 123 |

### Example 6: Synthesis (6) of compound represented by formula (III) (compound represented by formula (II) where R₁, R₂, and R₃ are all hydrogen) using compound represented by formula VI

A portion (8 mL) of the pH 3.3 aqueous sample solution prepared in Example 1 was adjusted to pH 4.6 by adding an aqueous sodium hydroxide solution, followed by heating for 1 hour with a water bath at 60°C. After that, the pH was adjusted to 10 with sodium hydroxide, and then the solution was further heated with a water bath at 90°C to prepare a heat-treated sample. The amount of Compound III in the obtained heat-treated sample was quantified.

### Example 7: Synthesis (7) of compound represented by formula (III) (compound represented by formula (II) where R₁, R₂, and R₃ are all hydrogen) using compound represented by formula VI

A portion (8 mL) of the pH 3.3 aqueous sample solution prepared in Example 1 was adjusted to pH 4.6 by adding an aqueous sodium hydroxide solution, followed by heating for 1 hour with a water bath at 90°C. After that, the pH was adjusted to 10 with sodium hydroxide, and then the solution was further heated with a water bath at 90°C to prepare a heat-treated sample. The amount of Compound III in the obtained heat-treated sample was quantified.

### Example 8: Synthesis (8) of compound represented by formula (III) (compound represented by formula (II) where R₁, R₂, and R₃ are all hydrogen) using compound represented by formula VI

γ-Glu-Cys-Cys(VI) synthesized with reference to Non Patent Literature 2 was dissolved in ultra pure water to prepare a pH 3.3 aqueous sample solution of a concentration of 106 ppm. To the pH 3.3 aqueous sample solution (3 mL), a 0.1 mM aqueous calcium chloride solution (3 mL) was added, and then the pH was adjusted to 4.6 with an aqueous sodium hydroxide solution. This aqueous solution was heated for 1 hour with a water bath at 60°C. To this heated aqueous solution (5 mL), a 5 mM aqueous EDTA solution (0.05 mL) was added, and then the pH was adjusted to 10 with sodium hydroxide. The solution was further heated with a water bath at 90°C to prepare a heat-treated sample. The amount of Compound III in the obtained heat-treated sample was quantified.

### Example 9: Synthesis (9) of compound represented by formula (III) (compound represented by formula (II) where R₁, R₂, and R₃ are all hydrogen) using compound represented by formula VI

To the pH 3.3 aqueous sample solution (3 mL) prepared in Example 5, an 0.6 mM aqueous calcium chloride solution (3 mL) was added, and then the pH was adjusted to 4.6 with an aqueous sodium hydroxide solution. This aqueous solution was heated for 1 hour with a water bath at 60°C. To the heated aqueous solution (5 mL), a 5 mM aqueous EDTA solution (0.05 mL) was added, and then the pH was adjusted to 10 with sodium hydroxide. The solution was further heated with a water bath at 90°C to prepare a heat-treated sample. The amount of Compound III in the obtained heat-treated sample was quantified.

The reaction yields were as follows.

**Table 3**

| | Yield of Compound III (%) |
|---|---|
| Example 6 | 13.6 |
| Example 7 | 13.2 |
| Example 8 | 18.5 |
| Example 9 | 26.1 |

### Example 10: Synthesis (10) of compound represented by formula (III) (compound represented by formula (II) where R₁, R₂, and R₃ are all hydrogen) using compound represented by formula V

Compound 26 obtained in Example 25 described later was dissolved in ultra pure water to prepare an aqueous solution of a concentration of 0.1 mM (154 ppm), and then a 26 mM aqueous TCEP solution (1 mL) was added. The pH of the obtained aqueous solution was adjusted to 8 by adding an aqueous sodium hydroxide solution. Then, the solution was heated for 12 hours with a water bath at 90°C to prepare a heat-treated sample. The amount of Compound III in the obtained heat-treated sample was quantified.

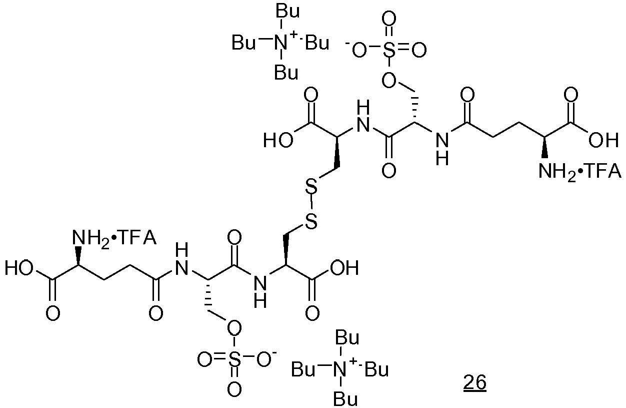

### Example 11: Synthesis (11) of compound represented by formula (III) (compound represented by formula (II) where R₁, R₂, and R₃ are all hydrogen) using compound represented by formula V

To a 0.1 mM aqueous solution of compound 26 prepared in Example 10, a 26 mM aqueous TCEP solution (1 mL) was added. The pH of the obtained aqueous solution was adjusted to 10 by adding an aqueous sodium hydroxide solution, and then the solution was heated for 4 hours with a water bath at 90°C to prepare a heat-treated sample. The amount of Compound III in the obtained heat-treated sample was quantified.

### Example 12: Synthesis (12) of compound represented by formula (III) (compound represented by formula (II) where R₁ ,R₂, and R₃ are all hydrogen) using compound represented by formula V

Compound 30 obtained in Example 29 described later was dissolved in ultra pure water to prepare an aqueous solution of a concentration of 0.1 mM (77 ppm), and then a 26 mM aqueous TCEP solution was added thereto. The pH of the obtained aqueous solution was adjusted to 10 by adding an aqueous sodium hydroxide solution, and then the solution was heated for 6 hours with a water bath at 90 °C to prepare a heat-treated sample. The amount of Compound III in the obtained heat-treated sample was quantified.

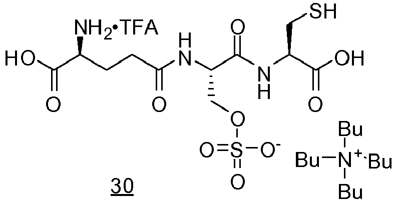

The reaction yields are as follows.

**Table 4**

| | Yield (%) |
|---|---|
| Example 10 | 7.9 |
| Example 11 | 42.1 |
| Example 12 | 43.4 |

The production method and the intermediate compound of the present invention make it possible to provide a lanthionine derivative which is extremely useful as a CaSR agonist, a kokumi-imparting agent, a food and/or beverage ingredient, and the like, and are extremely industrially useful.

### Example 13: Synthesis of 9 to 14, which are compounds represented by formula (V) and oxidative dimers thereof

To an acetonitrile solution (15 mL) of Boc-Glu-Ot-Bu (N-t-butoxycarbonylglutamic acid t-butyl ester) (455 mg, 1.50 mmol), HOAt (1-hydroxy-7-azabenzotriazole) (226 mg, 1.66 mmol) and WSC·HCl (315 mg, 1.64 mmol) were added, followed by stirring at room temperature for 1 hour. To the reaction liquid, H-Ser (t-Bu) -OMe (350 mg, 1.65 mmol) and triethylamine (0.3 mL, 2.15 mmol) were added, followed by stirring for further 15 hours. The reaction liquid was concentrated under reduced pressure to approximately 2 mL, and then ethyl acetate was added thereto. The organic layer was washed by partitioning with a 10% aqueous citric acid solution and then a saturated aqueous sodium hydrogen carbonate solution. The organic layer was dried over anhydrous sodium sulfate, and then concentrated to dryness under reduced pressure to obtain compound 9 (637 mg) as a colorless sticky substance. Here, Boc in the formula of compound 9 means t-butoxycarbonyl residue (-COO-t-Bu). Yield 92%; ESI MS m/z 461.2 (M+H)⁺; ¹H NMR (400 MHz, CD₃OD) δ 1.19 (9H, s), 1.47 (9H, s), 1.49 (9H, s), 1.88 (2H, m), 2.10 (2H, m), 2.38 (2H, t, J=7.6 Hz), 3.63 (1H, dd, J=3.6, 9.6 Hz), 3.75 (3H, s), 3.80 (1H, dd, J=4.4, 9.6 Hz), 4.00 (1H, m), 4.61 (1H, dd, J=3.6, 4.4 Hz).

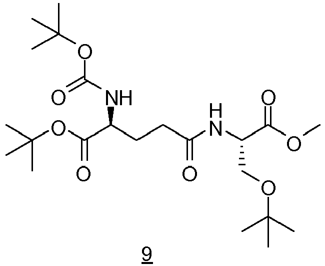

Compound 9 (620 mg, 1.35 mmol) was dissolved in methanol (5 mL), and then a 1 N aqueous sodium hydroxide solution (2.5 mL, 2.50 mmol) was added thereto under ice-cooling, followed by stirring at room temperature for 2 hours. The solution was concentrated under reduced pressure to approximately 3 mL, and then the pH was adjusted to a sufficiently acidic region by adding a 10% aqueous citric acid solution. Then, extraction was conducted by partitioning with ethyl acetate. The organic layer was dried over anhydrous sodium sulfate, and then was concentrated to dryness under reduced pressure to obtain compound 10 (578 mg) as a colorless sticky substance. Yield 96%; ESI MS m/z 447.2 (M+H)⁺; ¹H NMR (400 MHz, CD₃OD) δ 1.20 (9H, s), 1.47 (9H, s), 1.49 (9H, s), 1.89 (2H, m), 2.10 (2H, m), 2.39 (2H, t, J=7.6 Hz), 3.65 (1H, dd, J=3.6, 9.2 Hz), 3.82 (1H, dd, J=4.4, 9.2 Hz), 4.00 (1H, m), 4.57 (1H, dd, J=3.6, 4.4 Hz).

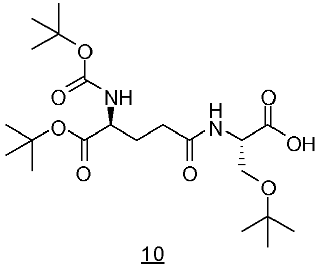

To an acetonitrile solution (10 mL) of compound 10 (357 mg, 0.80 mmol), HOAt (122 mg, 0.90 mmol) and WSC·HCl (170 mg, 0.89 mmol) were added, followed by stirring at room temperature for 1 hour. To the reaction liquid, (H-Cys-Ot-Bu)₂·2HCl (cystine t-butyl ester hydrochloride) (170 mg, 0.40 mmol) and triethylamine (0.2 mL, 1.43 mmol) were added, followed by stirring for further 15 hours. The reaction liquid was concentrated under reduced pressure to approximately 2 mL, and then ethyl acetate was added thereto. The organic layer was washed by partitioning with a 10% aqueous citric acid solution and then with a saturated aqueous sodium hydrogen carbonate solution. The organic layer was dried over anhydrous sodium sulfate, and then concentrated to dryness under reduced pressure to obtain compound 11 (441 mg) as a white solid. yield 91%; ESI MS m/z 1232.7 (M+Na)⁺; ¹H NMR (400 MHz, CD₃OD) δ 1.22 (18H, s), 1.47 (18H, s), 1.49 (18H, s), 1.50 (18H, s), 1.90 (2H, m), 2.11 (2H, m), 2.39 (4H, m), 3.06 (2H, m), 3.21 (2H, m), 3.64 (4H, m), 4.02 (2H, m), 4.55 (2H, m), 4.64 (2H, m).

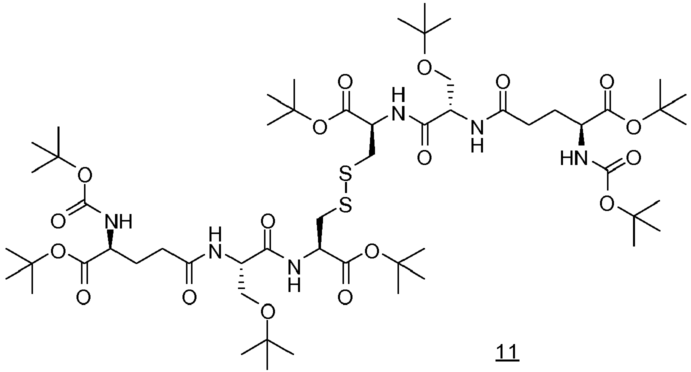

Compound 11 (82 mg, 0.068 mmol) was dissolved in a 4 N HCl-dioxane solution (2 mL), followed by stirring for 4 hours under ice-cooling. The solvent and the reagent in excess were removed under reduced pressure to obtain a hydrochloride of compound 12 as a white solid.
Yield 100%; ESI MS m/z 673.3 (M+H)⁺; ¹H NMR (400 MHz, CD₃OD) δ 2.21 (2H, m), 2.29 (2H, m), 2.64 (4H, m), 3.10 (2H, m), 3.30 (2H, m), 3.85 (4H, m), 4.10 (2H, m), 4.54 (2H, m), 4.77 (2H, m).

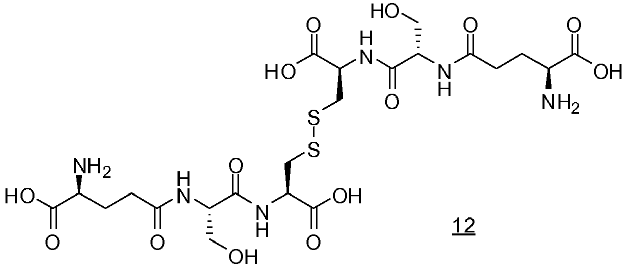

Compound 12 (100 mg, 0.083 mmol) was dissolved in a citrate buffer/methanol mixture solution (pH 5) (3 mL), and then TCEP·HCl (tris(2-carboxyethyl)phosphine hydrochloride (30 mg, 0.10 mmol) was added, followed by stirring at room temperature for 0.5 hours. To the reaction liquid, ethyl acetate was added, and then the liquid was washed by partitioning with a saturated aqueous sodium hydrogen carbonate solution. The organic layer was dried over anhydrous sodium sulfate, and then concentrated to dryness under reduced pressure to obtain compound 13 (82 mg). Yield 82%; ESI MS m/z 628.4 (M+Na)⁺; ¹H NMR (400 MHz, CD₃OD) δ 1.23 (9H, s), 1.47 (9H, s), 1.49 (9H, s), 1.50 (9H, s), 1.90 (1H, m), 2.14 (1H, m), 2.40 (2H, t, J=7.6 Hz), 2.91 (1H, dd, J=5.6, 14.0 Hz), 2.98 (1H, dd, J=4.8, 14.0 Hz), 3.63 (1H, dd, J=5.6, 8.8 Hz), 3.71 (1H, dd, J=4.8, 8.8 Hz), 4.01 (1H, m), 4.48-4.63 (2H, m).

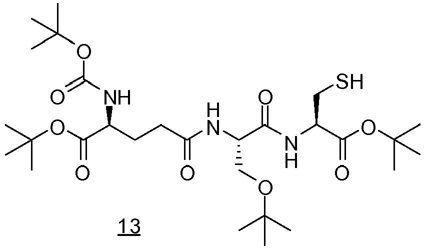

Compound 13 (59 mg, 0.097 mmol) was dissolved in a 4 N HCl-dioxane solution (2 mL), followed by stirring for 4 hours under ice-cooling. Thus, a hydrochloride of compound 14 was obtained as a white solid.
Yield 100%; ESI MS m/z 338.0 (M+H)⁺; ¹H NMR (400 MHz, D₂O) δ 2.12 (2H, m), 2.50 (2H, m), 2.89 (2H, m), 3.80 (2H, m), 3.85 (1H, m), 4.08 (1H, m), 4.56 (1H, m).

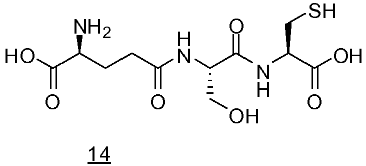

The thus obtained compound 14 can be used in the method for producing the compound represented by formula (II) or a chemically acceptable salt thereof, for example, by converting the hydroxy group to an activated ester such as a phosphate ester or a sulfate ester.

### Example 14: Synthesis of compound 15 which is oxidative dimer of compound represented by formula (V)

To a tetrahydrofuran solution (9 mL) of Boc-Glu-Ot-Bu (0.305 g, 1.01 mmol), triethylamine (0.21 mL, 1.51 mmol) and pivaloyl chloride (0.185 mL, 1.50 mmol) were added, followed by stirring at 0°C for 0.5 hours. To the reaction liquid, cystine (0.24 g, 1.00 mmol) and an aqueous triethylamine solution (0.28 mL, 2.01 mmol) were added, followed by stirring at 0°C for further 2 hours. The solution was concentrated under reduced pressure. Then, a 10% aqueous citric acid solution was added thereto, followed by extraction with ethyl acetate. The ethyl acetate layer was dried over anhydrous sodium sulfate, and then concentrated to dryness under reduced pressure. The residue was purified by fractionation using HPLC (column: XBridge manufactured by Waters Corporation, 1.9 i.d. x 5 cm, linear gradient of water/acetonitrile containing 0.1% formic acid) to obtain compound 15 (0.25 g) as a white solid.
Yield 61%; ESI MS m/z 811.0 (M+H)⁺; ¹H NMR (400 MHz, CD₃OD) δ1.46 (18H, s), 1.49 (18H, s), 1.90 (1H, m), 2.11 (2H, m), 2.38 (4H, m), 3.03 (2H, dd, J=8.8, 13.9 Hz), 3.29 (2H, dd, J=4.4, 13.9 Hz), 4.01 (2H, m), 4.75 (2H, dd, J=4.4, 8.8 Hz).

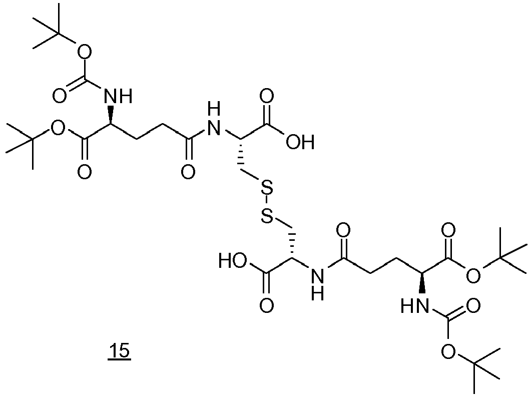

### Example 15: Synthesis of compound 16, which is oxidative dimer of compound represented by formula (V)

To a tetrahydrofuran solution (3 mL) of compound 15 (0.1 g, 0.12 mmol), pivaloyl chloride (46 µL, 0.37 mmol) and triethylamine (55 µL, 0.39 mmol) were added, followed by stirring for 0.5 hours under ice-cooling. To the reaction liquid, serine sulfuric acid ester (93 mg, 0.50 mmol), a 10% aqueous tetrabutylammonium hydroxide solution (1.4 mL, 0.54 mmol), and triethylamine (70 µL, 0.50 mmol) were added, followed by stirring for further 1 hour. To the reaction liquid, water was added, and then the pH was adjusted to 5 to 6 with a 10% aqueous citric acid solution, followed by extraction with n-butanol. The organic layer was concentrated to dryness under reduced pressure, and the obtained residue was purified by HPLC (column: XBridge manufactured by Waters Corporation, 1.9 i.d. x 5 cm, linear gradient of water/acetonitrile containing 0.1% formic acid) to obtain compound 16 (35 mg) as a colorless sticky substance.
Yield 17%; ESI MS m/z 1384 (M-Bu₄N)⁻; ¹H NMR (400 MHz, CD₃OD) δ1.04 (24H, t, J=7.2 Hz), 1.41-1.49 (52H, m), 1.68 (16H, m), 1.93 (2H, m), 2.15 (2H, m), 2.41 (4H, m), 2.96 (2H, dd, J=9.6, 14.0 Hz), 3.26 (18H, m), 4.00 (2H, dd, J=4.4, 9.6 Hz), 4.37 (4H, m), 4.67 (2H, t, J=3.2 Hz), 4.83 (2H, dd, J=4.4, 9.6 Hz).

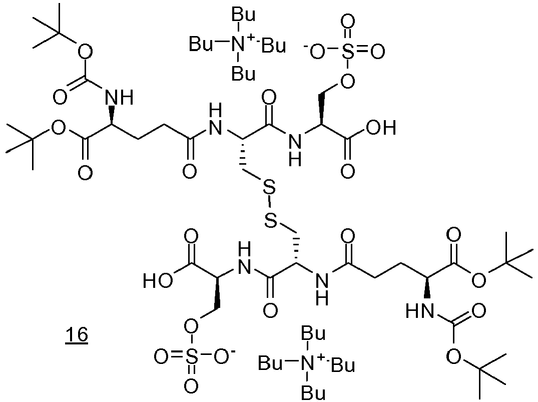

### Example 16: Synthesis of compound 17 represented by formula (V)

Compound 16 (35 mg, 0.021 mmol) was dissolved in methanol (0.2 mL), and water (0.1 mL) and TCEP (15 mg, 0.06 mmol) were added thereto, followed by stirring at room temperature for 0.5 hours. To the reaction liquid, water was added, followed by extraction with ethyl acetate. The organic layer was concentrated to dryness under reduced pressure to obtain compound 17 (28 mg) as a colorless sticky substance.
Yield 80%; ESI MS m/z 571.7 (M-Bu₄N)⁻ ; ¹H NMR (400 MHz, CD₃OD) δ1.05 (12H, t, J=7.2 Hz), 1.43 (8H, m), 1.47 (9H, s), 1.49 (9H, s), 1.69 (8H, m), 1.92 (1H, m), 2.14 (1H, m), 2.44 (2H, m), 2.87-2.99 (2H, m), 3.26 (8H, m), 4.00 (1H, dd, J=4.8, 8.8 Hz), 4.32-4.41 (2H, m), 4.66 (1H, m), 4.83 (1H, m).

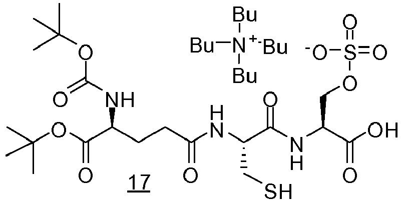

### Example 17: Synthesis of compound 18 represented by formula (V)

Compound 17 (25 mg, 0.031 mmol) was dissolved in a 95% aqueous trifluoroacetic acid solution (0.2 mL), followed by stirring for 2 hours under ice-cooling. The solution was concentrated to dryness under reduced pressure. The residue was purified by HPLC (column: Develosil RP-AQUEOUS-AR-5 manufactured by Nomura Chemical Co., Ltd, 1.0 i.d. x 25 cm, linear gradient of water/acetonitrile containing 0.1% heptafluorobutyric acid) to obtain compound 18 (3 mg) as a white solid.
Yield 23%; ESI MS m/z 415.9; ¹H NMR (400 MHz, D₂O) δ2.04 (2H, m), 2.45 (2H, m), 2.77-2.87 (2H, m), 3.72 (1H, t, J=6.4 Hz), 4.21 (1H, dd, J=3.2, 10.4 Hz), 4.28 (1H, dd, J=4.8, 10.4 Hz), 4.46 (1H, dd, J=5.6, 6.8 Hz), 4.56 (1H, dd, J=3.2, 4.8 Hz).

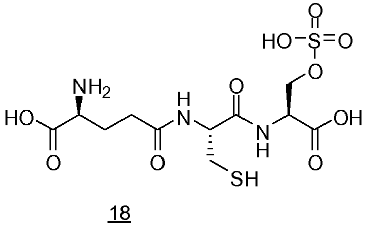

### Synthesis Example 18: Synthesis of compound 19

To a tetrahydrofuran solution (6 mL) of Boc-Glu-Ot-Bu (0.15 g, 0.49 mmol), triethylamine (0.105 mL, 0.75 mmol) and pivaloyl chloride (95 µL, 0.77 mmol) were added, followed by stirring at 0°C for 0.5 hours. To the reaction liquid, serine sulfuric acid ester (0.186 g, 1.01 mmol), a 10% aqueous tetrabutylammonium hydroxide solution (2.6 mL, 1.00 mmol), and triethylamine (0.14 mL, 1.01 mmol) were added, followed by stirring at 0°C for further 0.5 hours. The pH of the reaction liquid was adjusted to approximately 5 with a 10% aqueous citric acid solution, and the reaction liquid was concentrated under reduced pressure to approximately 10 mL, followed by extraction with n-butanol. The n-butanol layer was concentrated to dryness under reduced pressure, and then the residue was purified by HPLC (column: XBridge manufactured by Waters Corporation, 1.9 i.d. x 5 cm, linear gradient of water/acetonitrile containing 0.1% formic acid) to obtain compound 19 (0.227 g).
Yield 64%; ESI MS m/z 469.0 (M-Bu₄N)⁻; ¹H NMR (400 MHz, CD₃OD) δ1.04 (12H, m), 1.46 (26H, m), 1.69 (8H, m), 1.93 (1H, m), 2.10 (1H, m), 2.39 (2H, m), 3.26 (8H, m), 3.98 (1H, m), 4.29 (1H, m), 4.35 (1H, m), 4.66 (1H, m).

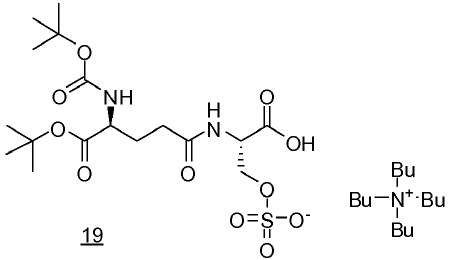

### Synthesis Example 19: Synthesis of compound 20

Compound 20 was synthesized by the same method as in Example 18, except that phosphoserine was used instead of serine sulfuric acid ester.
Yield 59%; ESI MS m/z 469.0 (M-Bu₄N)⁻; ¹H NMR (400 MHz, CD₃OD) δ 1.04 (12H, m), 1.46 (26H, m), 1.68 (8H, m), 1.93 (1H, m), 2.09 (1H, m), 2.40 (2H, m), 3.27 (8H, m), 3.98 (1H, m), 4.16 (1H, m), 4.27 (1H, m), 4.57 (1H, m).

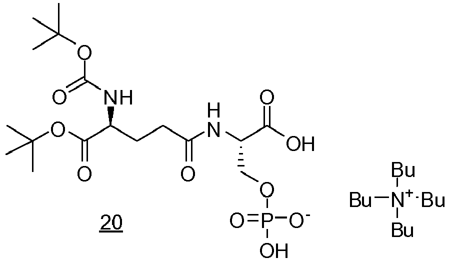

### Synthesis Example 20: Synthesis of compound 21

Compound 21 was synthesized by the same method as in Example 18, except that serine was used instead of serine sulfuric acid ester.
Yield 79%; m/z 413.0 (M+Na)⁺; ¹H NMR (400 MHz, CD₃OD) δ1.46 (9H, s), 1.49 (9H, s), 1.89 (1H, m), 2.13 (1H, m), 2.40 (2H, t, J=7.6 Hz), 3.85 (1H, dd, J=3.6, 11.2 Hz), 3.91 (1H, dd, J=4.8, 11.2 Hz), 4.00 (1H, dd, J=4.4, 6.9 Hz), 4.52 (1H, dd, J=3.6, 4.8 Hz).

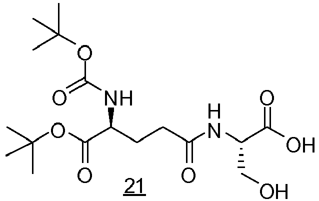

### Example 21: Synthesis of compound 22, which is oxidative dimer of compound represented by formula (V)

To a THF solution (3 mL) of compound 19 (0.11g, 0.15 mmol) obtained in Example 18, HOSu (N-hydroxysuccinimide) (18 mg, 0.16 mmol) and WSC·HCl (30 mg, 0.16 mmol) were added, followed by stirring for 1 hour. To the reaction liquid, (H-Cys-Ot-Bu)₂·2HCl (33 mg, 0.078 mmol) and an acetonitrile solution (2 mL) of triethylamine (22 µL, 0.16 mmol) were added, followed by stirring for further 2 hours. To a liquid concentrate obtained under reduced pressure, a 10% aqueous citric acid solution was added to adjust the pH to 5, followed by extraction with ethyl acetate. The organic layer was dried over anhydrous sodium sulfate, and then concentrated to dryness under reduced pressure. The residue was purified by HPLC (column: XBridge manufactured by Waters Corporation, 1.9 i.d. x 5 cm, linear gradient of water/acetonitrile containing 0.1% formic acid) to obtain compound 22 (26 mg).
Yield 19%; ESI MS m/z 1496.0 (M-Bu₄N)⁻; ¹H NMR (400 MHz, CD₃OD) δ1.45 (24H, m), 1.41-1.50 (70H, m), 1.67 (16H, m), 1.95 (2H, m), 2.11 (2H, m), 3.07 (2H, dd, J=8.0, 13.6 Hz), 3.21-3.28 (18H, m), 3.98 (2H, m), 4.27 (4H, m), 4.61 (2H, m), 4.68 (2H, m).

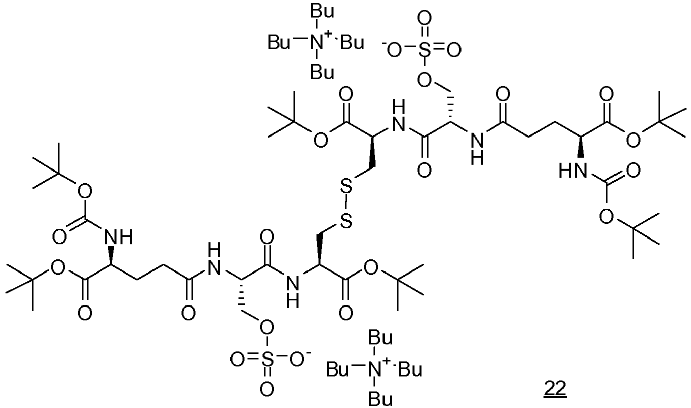

### Example 22: Synthesis of compound 23, which is oxidative dimer of compound represented by formula (V)

Compound 23 was obtained by the same reaction as in Example 21, except that compound 20 of Example 19 was used instead of compound 19.
Yield 11%; ESI MS m/z 1255.0 (M-2Bu₄N)⁻; ¹H NMR (400 MHz, CD₃OD) δ1.05 (24H, t, J=7.2 Hz), 1.41-1.49 (70H, m), 1.69 (16H, m), 1.92 (2H, m), 2.92 (2H, m), 2.41 (4H, m), 3.10 (2H, dd, J=9.2, 14.0 Hz), 3.26 (18H, m), 3.98 (2H, dd, J=4.8, 9.2 Hz), 4.16 (2H, m), 4.27 (2H, m), 4.57 (2H, m), 4.69 (2H, m).

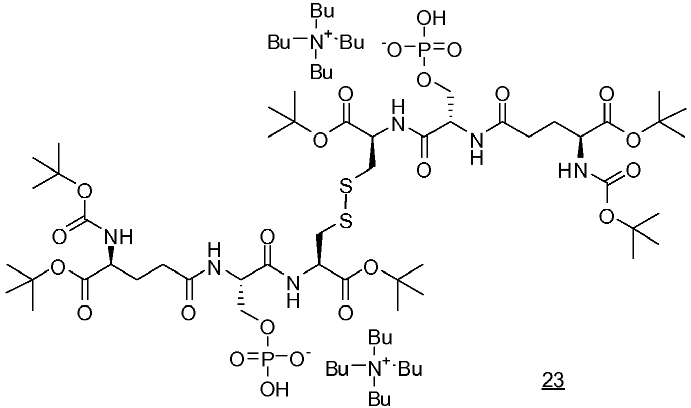

### Example 23: Synthesis of compound 24, which is oxidative dimer of compound represented by formula (V)

Compound 24 was synthesized by the same reaction as in Example 21, except that compound 21 of Example 20 was used instead of compound 19.
Yield 95%; ESI MS m/z 1119.2 (M+Na)⁺; ¹H NMR (400 MHz, CD₃OD) δ 1.47 (18H, s), 1.49 (36H, s), 1.91 (2H, m), 2.13 (2H, m), 2.41 (4H, m), 3.07 (2H, m), 3.23 (2H, m), 3.82 (4H, m), 4.00 (2H, m), 4.51 (2H, m), 4.64 (2H, m).

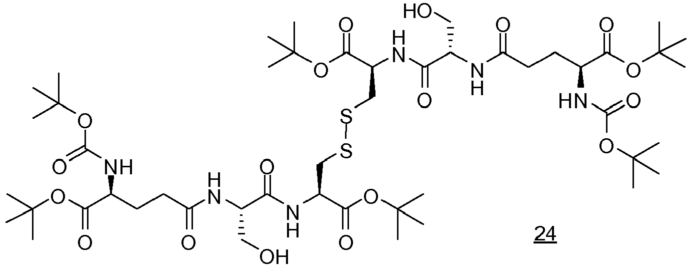

### Example 24: Synthesis of compound 25, which is oxidative dimer of compound represented by formula (V)

To a pyridine solution (0.2 mL) of compound 24 (40 mg, 0.1 mmol) of Example 23, acetic anhydride (0.1 mL) was added, followed by stirring for 2 hours under ice-cooling. The solvent and the reagent in excess were removed under reduced pressure, and then the residue was purified by fractionation using HPLC (column: XBridge manufactured by Waters Corporation, 1.9 i.d. x 5 cm, linear gradient of water/acetonitrile containing 0.1% formic acid) to obtain compound 25 (16 mg) as a white solid. Yield 37%; ESI MS m/z 1203.1 (M+Na)⁺; ¹H NMR (400 MHz, CD₃OD) δ1.46 (18H, s), 1.47 (36H, s), 1.90 (2H, m), 2.08 (6H, s), 2.10 (2H, m), 2.40 (4H, m), 3.02 (2H, m), 3.24 (2H, m), 4.00 (2H, m), 4.32 (2H, m), 4.39 (2H, m), 4.64 (2H, m), 4.76 (2H, m).

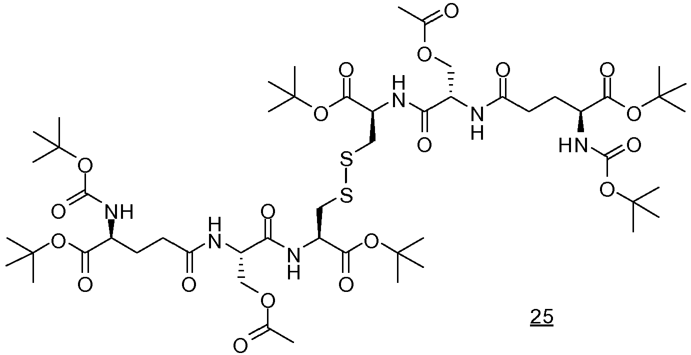

### Example 25: Synthesis of compound 26, which is oxidative dimer of compound represented by formula (V)

Compound 22 (5 mg, 0.0029 mmol) of Example 21 was dissolved in a 95% aqueous trifluoroacetic acid solution (0.2 mL), followed by stirring for 2 hours under ice-cooling. The solution was concentrated to dryness under reduced pressure to obtain compound 26 (4.5 mg). Here, TFA represents trifluoroacetic acid.
Yield 100%; ESI MS m/z 1072.0 (M-2TFA-Bu₄N)⁻; ¹H NMR (400 MHz, D₂O) δ1.05 (24H, t, J=7.6 Hz), 1.46 (16H, m), 1.75 (16H, m), 2.36 (4H, m), 2.73 (4H, m), 3.18 (2H, dd, J=8.8, 14.4 Hz), 3.30 (8H, m), 3.45 (2H, dd, J=4.4, 14.4 Hz), 4.22 (2H, t, J=6.4 Hz), 4.43 (2H, d, J=4.8 Hz), 4.86 (2H, t, J=4.8 Hz).

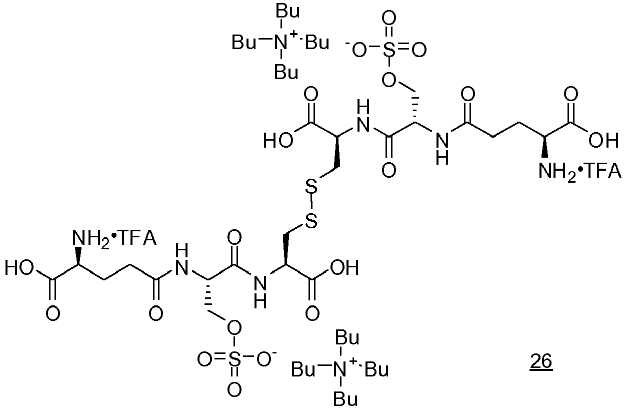

### Example 26: Synthesis of compound 27, which is oxidative dimer of compound represented by formula (V)

Compound 27 was obtained by the same reaction as in Example 25, except that compound 25 of Example 24 was used instead of compound 23 of Example 22.
ESI MS m/z 756.9 (M+H)⁺; ¹H NMR (400 MHz, CD₃OD) δ2.08 (6H, s), 2.19 (4H, m), 2.60 (4H, m), 3.05 (2H, m), 3.25 (2H, m), 4.03 (2H, m), 4.36 (4H, m), 4.76 (4H, m).

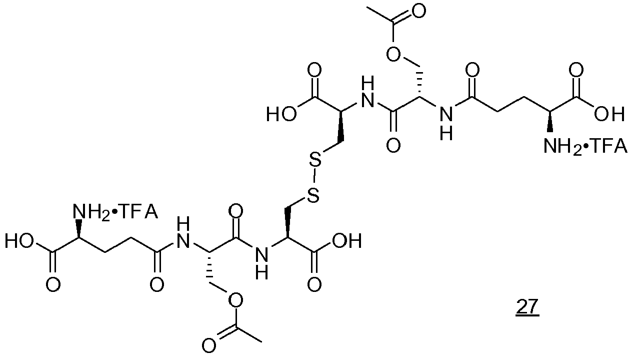

### Example 27: Synthesis of compound 28 represented by formula (V)

Compound 22 (5 mg, 0.0029 mmol) of Example 21 was dissolved in methanol (0.2 mL), and then water (0.5 mL) and TCEP (2 mg, 0.008 mmol) were added thereto, followed by stirring at room temperature for 10 minutes. To the reaction liquid, water was added, followed by extraction with ethyl acetate. Then, the extract was concentrated to dryness under reduced pressure to obtain compound 28 (4 mg) as a colorless sticky substance. Yield 80%; ESI MS m/z 627.9 (M-Bu₄N)⁻; ¹H NMR (400 MHz, CD₃OD) δ1.05 (12H, t, J=7.2 Hz), 1.44 (8H, m), 1.50 (9H, s), 1.56 (18H, s), 1.68 (8H, m), 1.93 (1H, m), 2.13 (1H, m), 2.42 (2H, m), 3.04 (1H, m), 3.25 (9H, m), 3.99 (1H, m), 4.25-4.31 (2H, m), 4.54-4.64 (2H, m).

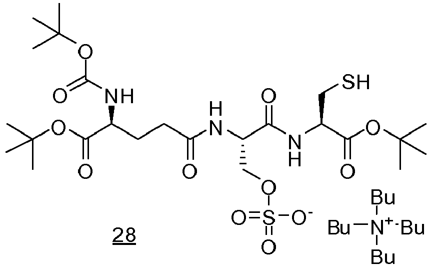

### Example 28: Synthesis of compound 29 represented by formula (V)

Compound 29 was obtained by the same method as in Example 27, except that compound 25 of Example 24 was used instead of compound 22.
Yield 77%; ESI MS m/z 592.0 (M+H)⁺; ¹H NMR (400 MHz, CD₃OD) δ1.47 (9H, s), 1.49 (9H, s), 1.50 (9H, s), 1.87 (1H, m), 2.08 (3H, s), 2.10 (1H, m), 2.39 (2H, t, J=7.2 Hz), 2.90 (1H, dd, J=6.4, 14.0 Hz), 2.97 (1H, dd, J=4.8, 14.0 Hz), 4.02 (1H, m), 4.30 (1H, dd, J=6.4, 11.2 Hz), 4.37 (1H, dd, J=4.8, 11.2 Hz), 4.53 (1H, dd, J=4.8, 6.4 Hz), 4.72 (1H, dd, J=4.8, 6.4 Hz).

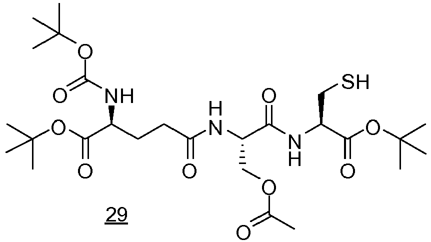

### Example 29: Synthesis of compound 30 represented by formula (V)

Compound 28 (4 mg, 0.0046 mmol) of Example 27 was dissolved in a 95% aqueous trifluoroacetic acid solution (0.2 mL), followed by stirring for 2 hours under ice-cooling. The solution was concentrated to dryness under reduced pressure to obtain compound 30 (3.5 mg).
Yield 99%; ESI MS m/z 415.9 (M-TFA-Bu4N)⁻; ¹H NMR (400 MHz, CD₃OD) δ1.05 (12H, t, J=7.2 Hz), 1.41 (8H, m), 1.67 (8H, m), 2.18 (1H, m), 2.28 (1H, m), 2.61 (2H, t, J=7.2 Hz), 2.92 (1H, dd, J=6.0, 14.0 Hz), 3.03 (1H, dd, J=4.4, 14.0 Hz), 4.07 (1H, t, J=6.4 Hz), 4.26 (1H, dd, J=6.8, 11.2 Hz), 4.33 (1H, dd, J=3.6, 11.2 Hz), 4.66 (1H, dd, J=4.4, 6.0 Hz), 4.70 (1H, dd, J=3.6, 6.4 Hz).

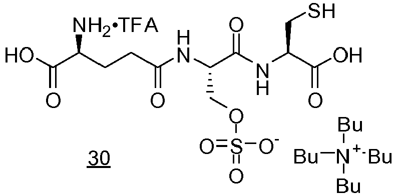

### Example 30: Synthesis of compound 31 represented by formula (V)

Compound 31 (8 mg) was obtained by the same method as in Example 29, except that compound 29 of Example 28 was used instead of compound 28.
Yield 96%; ESI MS m/z 377.8 (M-H)⁻ ; ¹H NMR (400 MHz, D₂O) δ2.08 (3H, s), 2.19 (2H, m), 2.60 (2H, m), 2.92-3.11 (2H, m), 3.84 (1H, m), 4.04-4.08 (2H, m), 4.64 (1H, m), 4.74 (1H, m).

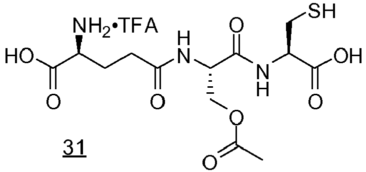

## Claims

1. A method for producing a compound represented by formula (II) or a chemically acceptable salt thereof, the method comprising a step of converting, by a reaction under an alkaline condition, a compound represented by the following general formula (I) or a chemically acceptable salt thereof to the compound (II) or the chemically acceptable salt thereof: 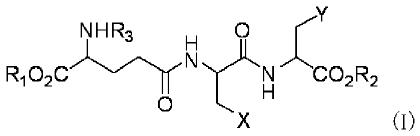 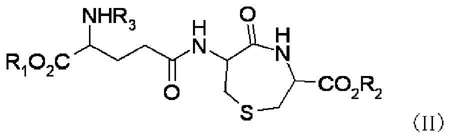
wherein R₁ and R₂, which may be the same or different, each represents a hydrogen atom or an optionally substituted lower alkyl group,
R₃ represents a hydrogen atom, an optionally substituted alkoxycarbonyl group, an optionally substituted sulfonyl group, or an acyl group,
X represents SR₄, OR₅, or a halogen atom, and Y represents SR₆, OR₇, or a halogen atom, provided that X and Y may be the same or different and at least one of X and Y is SR₄ or SR₆,
R₄ and R₆, which may be the same or different, each represents a hydrogen atom, an acyl group, an optionally substituted lower alkyl group, PO₃H₂, or SO₃H, and
R₅ and R₇, which may be the same or different, each represents an acyl group, an optionally substituted lower alkyl group, PO₃H₂, or SO₃H.

2. The production method according to claim 1, wherein
X is SR₄, and Y is OR₇.

3. The production method according to claim 1, wherein
X is OR₅, and Y is SR₆.

4. The production method according to claim 1, wherein
X is SR₄, and Y is SR₆.

5. The production method according to claim 1, wherein
X and Y, which may be the same or different, each represents SH, O-PO₃H₂, or O-SO₃H, provided that at least one of X and Y represents SH.

6. The production method according to claim 1, wherein
X is SR₄, Y is SR₆, and one of R₄ and R₆ is a hydrogen atom.

7. The production method according to claim 1, wherein
X is SR₄, Y is SR₆, and R₄ and R₆ are both hydrogen atoms.

8. A method for producing a compound represented by formula (II) or a chemically acceptable salt thereof, the method comprising a step of converting, by a cyclization reaction, a compound represented by the following general formula (I-2) or a chemically acceptable salt thereof to the compound (II) or the chemically acceptable salt thereof: 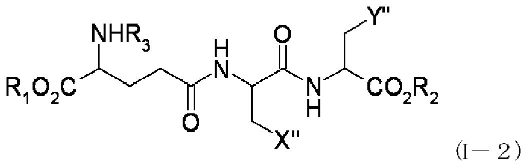 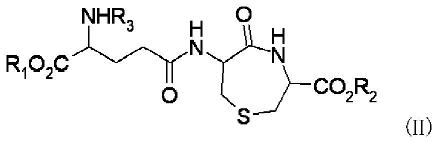
wherein R₁ and R₂, which may be the same or different, each represents a hydrogen atom or an optionally substituted lower alkyl group,
R₃ represents a hydrogen atom, an optionally substituted alkoxycarbonyl group, an optionally substituted sulfonyl group, or an acyl group,
X" and Y" each represents SH, SR₄, OR₅, or a halogen atom, provided that one of X'' and Y'' is SH, and the other one is not SH, and
R₄ and R₅, which may be the same or different, each represents an acyl group, an optionally substituted lower alkyl group, PO₃H₂, or SO₃H.

9. A method for producing a compound represented by compound (III) or a chemically acceptable salt thereof, the method comprising converting, by a reaction under an alkaline condition, a compound represented by the following formula (IV) or a chemically acceptable salt thereof to the compound (III) or the chemically acceptable salt thereof:

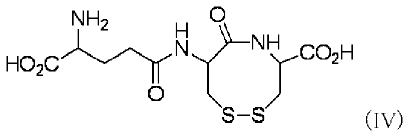

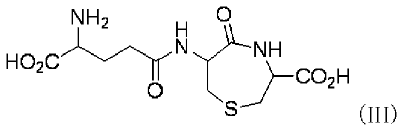

10. A method for producing a compound represented by formula (III) or a chemically acceptable salt thereof in a case where R₁, R₂ and/or R₃ in the compound represented by formula (II) according to any one of claims 1 to 8 are/is a group(s) other than a hydrogen atom, the method comprising a step of removing a protective group(s), which is(are) the group(s) other than a hydrogen atom, from the compound represented by formula (II) or a chemically acceptable salt thereof:

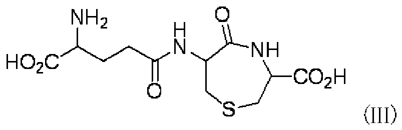

11. A compound represented by the following formula (V) or a chemically acceptable salt thereof: 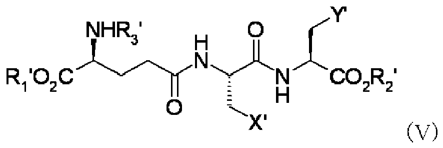
wherein R₁' and R₂', which may be the same or different, each represents a hydrogen atom or an optionally substituted lower alkyl group,
R₃' represents a hydrogen atom, an optionally substituted alkoxycarbonyl group, or an optionally substituted sulfonyl group, and
one of X' and Y' is SH, and the other one is OR₅', where R₅' represents a hydrogen atoms, an acyl group, an optionally substituted lower alkyl group, PO₃H₂, or SO₃H, provided that when X' is SH, Y' is not OH.

12. The compound according to claim 11 or a chemically acceptable salt thereof, wherein
the compound according to claim 11 is γ-Glu-Ser-Cys.

13. Any one of the following compounds or a chemically acceptable salt thereof: 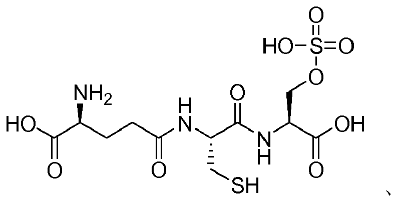 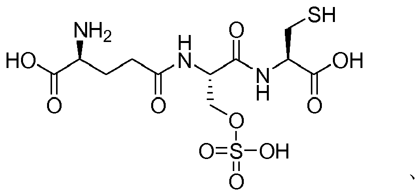 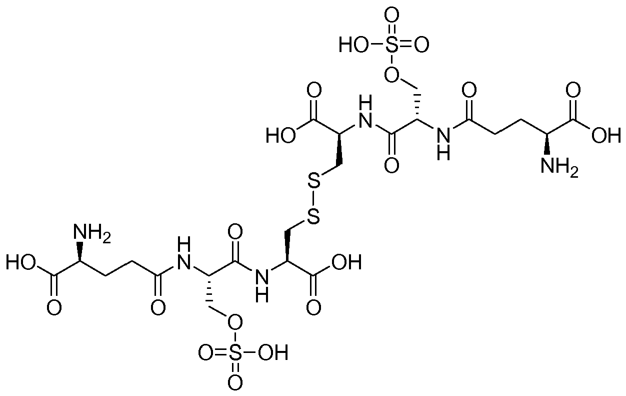 , and

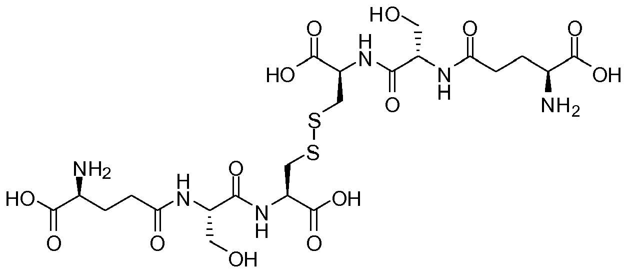

14. Use of the compound according to any one of claims 11, 12, and 13 or a chemically acceptable salt thereof for production of a CaSR agonist.

15. Use of the compound according to any one of claims 11, 12, and 13 or a chemically acceptable salt thereof for production of a kokumi-imparting agent.

16. Use of the compound according to any one of claims 11, 12, and 13 or a chemically acceptable salt thereof for production of a food and/or beverage ingredient.

## Amended claims

### Amended claims under Art. 19.1 PCT

**1.** A method for producing a compound represented by formula (II) or a chemically acceptable salt thereof, the method comprising a step of converting, by a reaction under an alkaline condition, a compound represented by the following general formula (I) or a chemically acceptable salt thereof to the compound (II) or the chemically acceptable salt thereof: 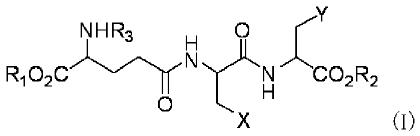 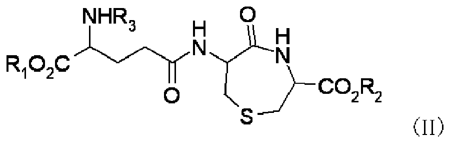
wherein R₁ and R₂, which may be the same or different, each represent a hydrogen atom or an optionally substituted lower alkyl group,
R₃ represents a hydrogen atom, an optionally substituted alkoxycarbonyl group, an optionally substituted sulfonyl group, or an acyl group,
X represents SR₄, OR₅, or a halogen atom, and Y represents SR₆, OR₇, or a halogen atom, provided that X and Y may be the same or different and at least one of X and Y is SR₄ or SR₆,
R₄ and R₆, which may be the same or different, each represent a hydrogen atom, an acyl group, an optionally substituted lower alkyl group, PO₃H₂, or SO₃H, and
R₅ and R₇, which may be the same or different, each represent an acyl group, an optionally substituted lower alkyl group, PO₃H₂, or SO₃H.

**2.** The production method according to claim 1, wherein X is SR₄, and Y is OR₇.

**3.** The production method according to claim 1, wherein
X is OR₅, and Y is SR₆.

**4.** The production method according to claim 1, wherein
X is SR₄, and Y is SR₆.

**5.** The production method according to claim 1, wherein X and Y, which may be the same or different, each represent SH, O-PO₃H₂, or O-SO₃H, provided that at least one of X and Y represents SH.

**6.** The production method according to claim 1, wherein
X is SR₄, Y is SR₆, and one of R₄ and R₆ is a hydrogen atom.

**7.** The production method according to claim 1, wherein
X is SR₄, Y is SR₆, and R₄ and R₆ are both hydrogen atoms.

**8.** A method for producing a compound represented by formula (II) or a chemically acceptable salt thereof, the method comprising a step of converting, by a cyclization reaction, a compound represented by the following general formula (I-2) or a chemically acceptable salt thereof to the compound (II) or the chemically acceptable salt thereof: 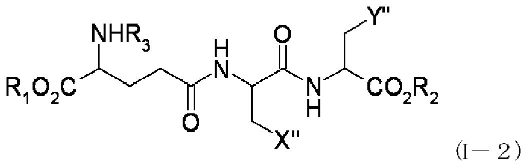 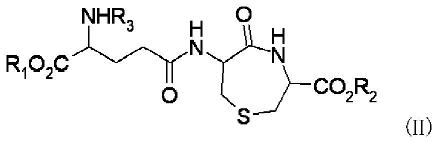
wherein R₁ and R₂, which may be the same or different, each represent a hydrogen atom or an optionally substituted lower alkyl group,
R₃ represents a hydrogen atom, an optionally substituted alkoxycarbonyl group, an optionally substituted sulfonyl group, or an acyl group,
X" and Y" reach represent SH, SR₄, OR₅, or a halogen atom, provided that one of X'' and Y'' is SH, and the other one is not SH, and
R₄ and R₅, which may be the same or different, each represent an acyl group, an optionally substituted lower alkyl group, PO₃H₂, or SO₃H.

**9.** A method for producing a compound represented by compound (III) or a chemically acceptable salt thereof, the method comprising converting, by a reaction under an alkaline condition, a compound represented by the following formula (IV) or a chemically acceptable salt thereof to the compound (III) or the chemically acceptable salt thereof:

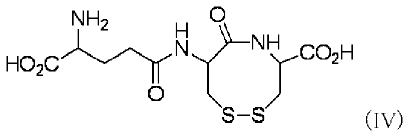

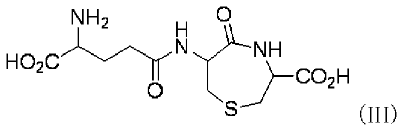

**10.** A method for producing a compound represented by formula (III) or a chemically acceptable salt thereof in a case where R₁, R₂ and/or R₃ in the compound represented by formula (II) according to any one of claims 1 to 8 are/is a group(s) other than a hydrogen atom, the method comprising a step of removing a protective group(s), which is(are) the group(s) other than a hydrogen atom, from the compound represented by formula (II) or a chemically acceptable salt thereof:

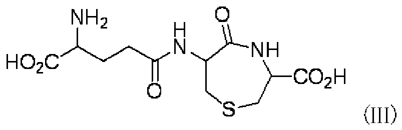

**11.** A compound represented by the following formula (V) or a chemically acceptable salt thereof: 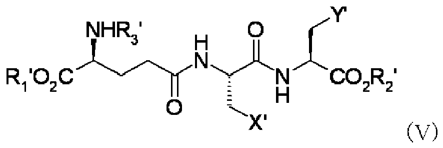
wherein R₁' and R₂', which may be the same or different, each represent a hydrogen atom or an optionally substituted lower alkyl group,
R₃' represents a hydrogen atom, an optionally substituted alkoxycarbonyl group, or an optionally substituted sulfonyl group, and
one of X' and Y' is SH, and the other one is OR₅', where R₅' represents a hydrogen atoms, an acyl group, an optionally substituted lower alkyl group, PO₃H₂, or SO₃H, provided that when X' is SH, Y' is not OH.

**12.** The compound according to claim 11 or a chemically acceptable salt thereof, wherein
the compound according to claim 11 is γ-Glu-Ser-Cys.

**13.** Any one of the following compounds or a chemically acceptable salt thereof: 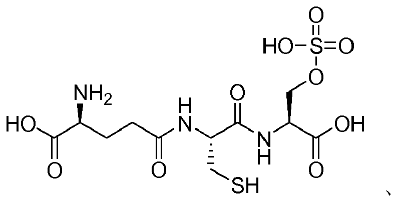 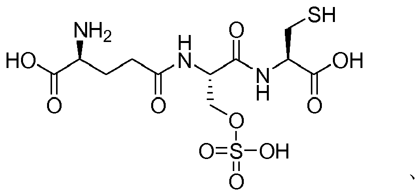 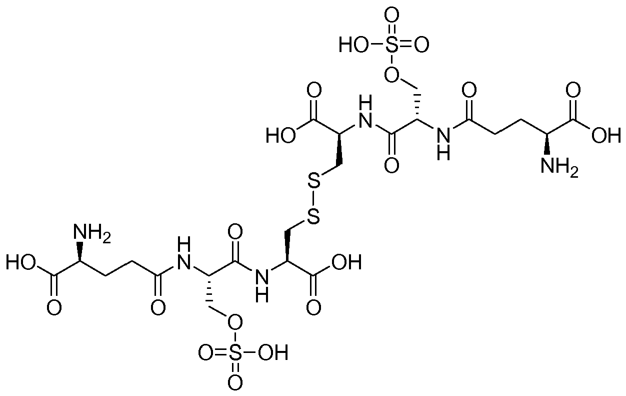 , and

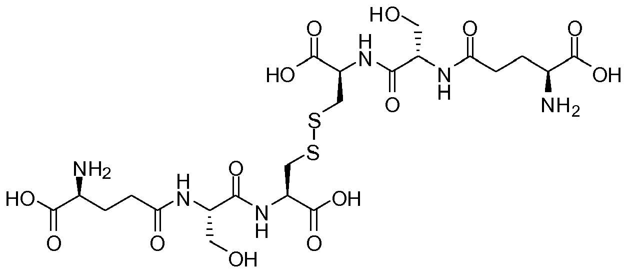

**14.** Use of the compound according to any one of claims 11, 12, and 13 or a chemically acceptable salt thereof for production of a CaSR agonist.

**15.** Use of the compound according to any one of claims 11, 12, and 13 or a chemically acceptable salt thereof for production of a kokumi-imparting agent.

**16.** Use of the compound according to any one of claims 11, 12, and 13 or a chemically acceptable salt thereof for production of a food and/or beverage ingredient.
